# EUROPEAN PATENT SPECIFICATION

(11) **EP 3 420 091 B1**
(45) Date of publication and mention of the grant of the patent: **28.07.2021**
(21) Application number: 17708317.7
(22) Date of filing: 24.02.2017
(51) Int. Cl.: C12N 15/86

(54) **NUCLEIC ACID CONSTRUCTS FOR PRODUCING RETROVIRAL VECTORS**
NUKLEINSÄUREKONSTRUKTE ZUR HERSTELLUNG RETROVIRALER VEKTOREN
CONSTRUCTIONS D'ACIDE NUCLÉIQUE POUR LA PRODUCTION DE VECTEURS RÉTROVIRAUX

(30) Priority: 26.02.2016 GB 201603372
(43) Date of publication of application: 02.01.2019
(73) Proprietor: UCL Business Ltd, London W1T 4TP (GB)
(72) Inventor: PULÉ, Martin, London W1T 4TP (GB); MEKKAOUI, Leila, London W1T 4TP (GB)
(74) Representative: D Young & Co LLP
(86) International application number: PCT/GB2017/050481
(87) International publication number: WO 2017/144892

(56) References cited:
- WO-A1-2014/121005
- WO-A1-2016/030690
- WO-A2-92/05266

## Description

### FIELD OF THE INVENTION

The present invention relates to the field of retroviral vectors. In particular, the invention relates to nucleic acid constructs and methods for producing retroviral vectors and to packaging cells and producer cells for use in such methods.

### BACKGROUND TO THE INVENTION

Retroviral vectors are relevant for a range of applications, including gene therapy. However, progress in lentiviral gene therapy, for example, has been hampered by the requirement for production of purified lentiviral vectors with high titre.

Methods for generating retroviral packaging cell lines are known in the art (see WO 92/05266, for example). Such packaging cell lines may be used to create producer cell lines for the production of retroviral vector particles.

Lentiviral production may be performed using transient transfection of 293T cells with four plasmids: the viral genome vector plasmid and three helper plasmids which supply Gagpol, Rev and Env glycoprotein. However, transient transfection technology cannot be used to generate virus at an appropriate scale for clinical applications because very large scale transfections are not practicable.

Stable lentiviral packaging cell lines can be generated by stably integrating all four of the above mentioned plasmids into a cell line (e.g. a 293T cell line) by transient transfection, followed by a serendipitous integration event. Cells which have integrated the plasmid are selected by the co-expression of an antibiotic resistance gene from the integrated plasmid. This approach is limited for a number of reasons. Firstly, stable integration following transient transfection is very inefficient. Second, antibiotic selection works well for a limited range of antibiotics, for example Zeocin™ and puromycin, but options are limited when attempting to select for the integration of four different genes and the available selection systems are not optimal. Thirdly, it is difficult to determine the relative expression obtained from each individual integration event.

Thus there is a need for retroviral, in particular lentiviral, production methods which are not associated with the disadvantages outlined above.

### SUMMARY OF ASPECTS OF THE INVENTION

The present inventors provide a retroviral production system which is based on the co-expression of viral proteins and detectable marker proteins which can be quantified at the level of a single cell. In particular, the system enables the expression level of different retroviral proteins in packaging cells to be tracked and packaging cells with ideal expression ratios for different retroviral components to be selected. For example, the expression level of the detectable marker may be determined using flow cytometry to identify and select packaging cells. Because the marker protein is co-expressed with the viral protein, packaging cell which express a particular viral protein or a combination of viral proteins at levels or ratios which are advantageous for a retroviral production system can be identified and selected.

In a first aspect the present invention provides a nucleic acid construct comprising:
(i) a first nucleic acid sequence which encodes a retroviral protein; and
(ii) a second nucleic acid sequence which encodes a detectable marker which is a cell surface protein comprising an extracellular domain and a membrane targeting domain.

The nucleic acid construct has the following structure:

A-X-B

in which
A is the first nucleic acid sequence which encodes a retroviral protein,
B is the second nucleic acid sequence encoding a detectable marker which is a cell surface protein comprising an extracellular domain and a membrane targeting domain and
X is a co-expression sequence which enables the retroviral protein and the detectable marker to be expressed as separate polypeptides.

The co-expression sequence may comprise an IRES sequence or a sequence encoding a self-cleaving peptide.

The co-expression sequence may be selected from a 2A self-cleaving peptide from an aphtho- or a cardiovirus, a 2A-like peptide, an internal ribosome entry site, a furin cleavage site or a Tobacco Etch Virus cleavage site.

The co-expression sequence may be a 2A self-cleaving peptide from an aphtho- or a cardiovirus or a 2A-like peptide.

The retroviral protein may be selected from gag-pol, env or rev.

The cell surface protein may be less than 200 amino acids.

The membrane targeting domain of the cell surface protein may comprise (i) a transmembrane domain and an endodomain; or (ii) a GPI anchor. The transmembrane domain may comprise a CD8 stalk or a part thereof.

The extracellular domain of the cell surface protein may comprise HA, V5, RQR8 or MYC
The cell surface protein may comprise an amino acid sequence shown as SEQ ID NO: 21-26 or a variant thereof which has at least 80% sequence identity.

The nucleic acid construct may further comprise transposon sequences flanking the A-X-B nucleic acid sequence. The transposon sequences may comprise the sequence shown as SEQ ID NO: 60 and 61 or a variant thereof sharing at least 80% sequence identity.

In a second aspect the present invention provides a kit comprising a plurality of nucleic acid constructs according to the present invention.

The kit may comprise:
(i) a first nucleic acid construct comprising a *gag-pol* sequence and a sequence encoding a first detectable marker;
(ii) a second nucleic acid construct comprising an *env* sequence and a sequence encoding a second detectable marker;
in which the first and second detectable markers are cell surface proteins comprising an extracellular domain and a membrane targeting domain and are different to each other.

The kit may further comprise:
(iii) a third nucleic acid construct comprising a *rev* sequence and a sequence encoding a third detectable marker which is a cell surface protein comprising an extracellular domain and a membrane targeting domain;
in which the first, second and third and detectable markers are all different to each other.

The kit may also comprise:
(iv) a fourth nucleic acid construct comprising a retroviral transfer vector and a sequence encoding a fourth detectable marker which is a cell surface protein comprising an extracellular domain and a membrane targeting domain;
in which the first, second, third (if present) and fourth detectable markers are all different to each other.

In a third aspect the present invention provides a plasmid comprising a nucleic acid construct of the present invention.

In a fourth aspect the present invention provides a packaging cell comprising a nucleic acid sequence of the invention which expresses at least one detectable marker which is a cell surface protein comprising an extracellular domain and a membrane targeting domain.

The term "co-expresses" means that the retroviral protein and the detectable marker are expressed together or at the same time. As a result, the expression level of the retroviral protein can be determined indirectly by assaying the expression level of the detectable marker with which it is co-expressed.

The packaging cell may co-express each of gag-pol, env and optionally rev with a different detectable marker.

The packaging cell may comprise:
(i) a first nucleic acid construct comprising a *gag-pol* sequence and a sequence encoding a first detectable marker;
(ii) a second nucleic acid construct comprising an *env* sequence and a sequence encoding a second detectable marker;
which co-expresses the first and second detectable markers at the cell surface.

The packaging cell may further comprise:
(iii) a third nucleic acid construct comprising a *rev* sequence and a sequence encoding a third detectable marker;
which co-expresses the first, second and third detectable markers at the cell surface.

In a fourth aspect the present invention provides a producer cell which comprises:
(i) a first nucleic acid construct comprising a *gag-pol* sequence and a sequence encoding a first detectable marker;
(ii) a second nucleic acid construct comprising an *env* sequence and a sequence encoding a second detectable marker;
(iii) optionally a third nucleic acid construct comprising a *rev* sequence and a sequence encoding a third detectable marker;
(iii) a fourth nucleic acid construct comprising a retroviral transfer vector and a sequence encoding a fourth detectable marker;
which co-expresses the first, second, third (if present) and fourth detectable markers at the cell surface.

The nucleic acid construct(s) may be stably integrated into the cell genome.

The packaging or producer cell may be a HEK293, HEK293-T, TE671 or HT1080, 3T3, or K562 cell.

In another aspect the present invention provides a method for making a packaging cell or producer cell of the invention which comprises the step of introducing one or more nucleic acid construct(s) of the invention, one or more plasmid(s) of the invention, a kit of nucleic acid constructs of the invention, into a cell.

In another aspect the present invention provides a method for selecting a packaging or producer cell of the invention by selecting for expression of the or each detectable marker encoded by the or each nucleic acid construct, wherein cells with optimal expression ratios of different retroviral components are selected from a population of cells.

The cell may express a plurality of detectable markers and may be selected using multiparameter flow cytometry.

The method may comprise the following steps:
(a) introducing
   (i) a first nucleic acid construct comprising a *gag-pol* sequence and a sequence encoding a first detectable marker;
   (ii) a second nucleic acid construct comprising an *env* sequence and a sequence encoding a second detectable marker;
   into a plurality of cells; and
(b) selecting a cell which co-expresses the first and second detectable markers.

Step (a) may further comprise introducing into the plurality of cells:
(iii) a third nucleic acid construct comprising a *rev* sequence and a sequence encoding a third detectable marker;
and step (b) further comprises selecting a cell which co-expresses the first, second and third detectable markers.

Step (a) may further comprise introducing into the plurality of cells:
(iv) a fourth nucleic acid construct comprising a retroviral transfer vector and a sequence encoding a fourth detectable marker;
and step (b) further comprises selecting a cell which co-expresses the first, second, fourth and optionally third detectable markers.

Step (a) may also comprise introducing a nucleic acid sequence encoding piggyBAC transposase.

In another aspect the invention provides a method for producing a retroviral vector which comprises the steps of culturing a producer cell of the present invention and isolating the retroviral vector.

In another aspect the invention provides a method for producing a retroviral vector which comprises the steps of introducing a retroviral vector genome into a packaging cell according to the present invention and isolating the retroviral vector.

The retroviral vector may be a lentiviral vector.

### DESCRIPTION OF THE FIGURES

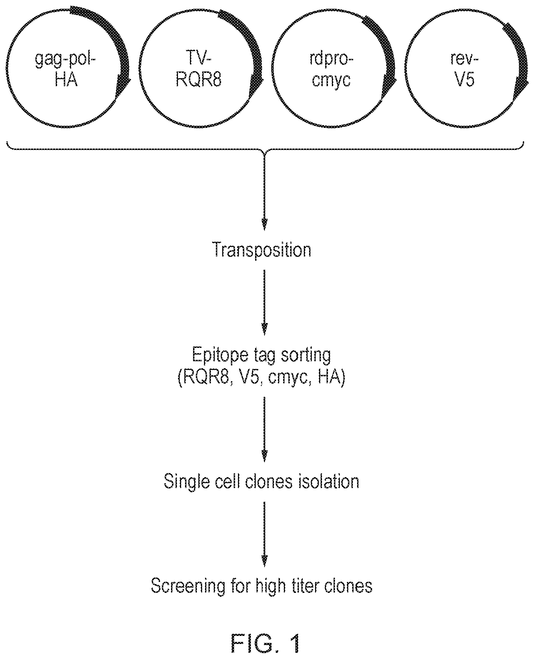

**Figure 1** shows a scheme for generation of a stable lentiviral producer line. Helper plasmids supplying gagpol, envelope (in this case RD-Pro) and rev also co-express surface expressed epitope tags (HA-tag, myc-tag and V5-tag respectively). One of the transfer vector transgenes is RQR8 which, like the tags, is easily detected on the surface of the packaging cell. In addition, all 4 of these expression cassettes are flanked by piggyBAC terminal repeats. Plasmids are transfected into 293T cells, along with an expression plasmid supplying the piggyBAC transposase. The transfer and helper cassettes are hence transposed into the 293T cells. After a period of culture, permanent expression of helper and transgene cassettes can easily be detected by flow cytometry. Large numbers of these "bulk" transposed cells are sorted by flow cytometric sorting, with precise population(s) of cells with high transgene and optimal relative expression being selected. These are directly sorted as single-cells by the flow-sorter. They are allowed to expand to fill the well of a 96 or 385 well plate and supernatant is titered. The highest titer clones are selected for virus production.

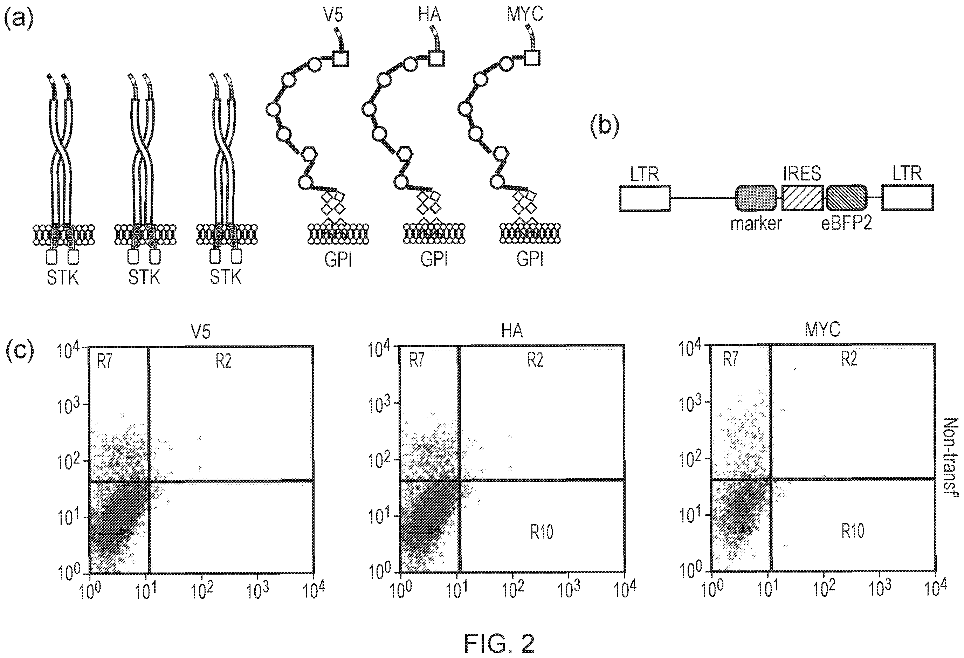

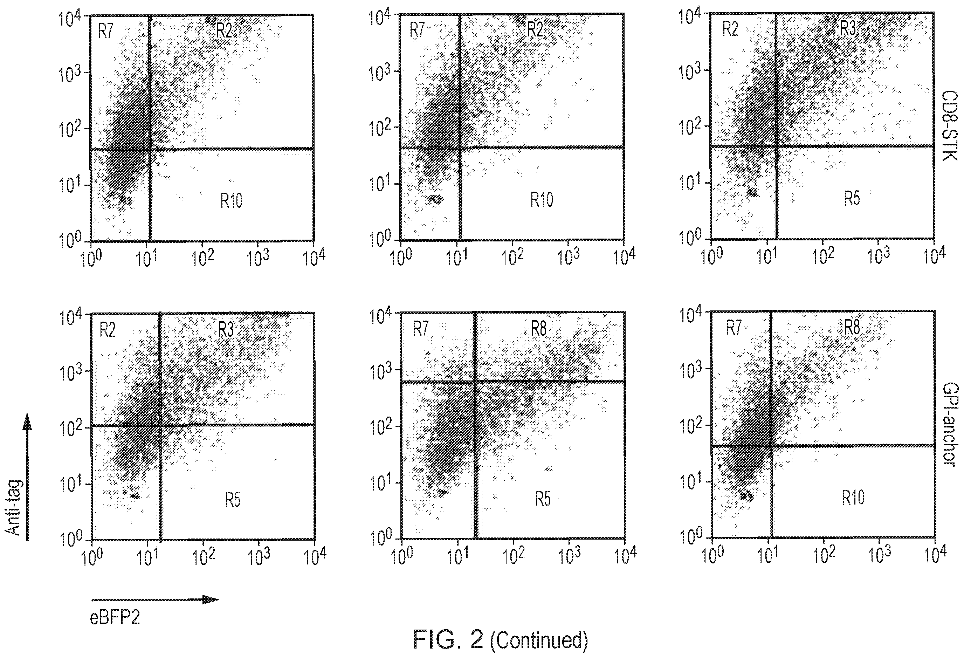

**Figure 2** shows the type of compact surface marker genes generated. (a) V5, HA and MYC-tags are expressed on a CD8-stalk and transmembrane domain. A small portion of the endodomain of CD8 is retained which contains polar residues which anchor the transmembrane protein and prevent it from being secreted from the membrane. An alternative strategy is to GPI-anchor the tags - with V5, HA and MYC-tags being shown attached to a GPI anchor. Here, the tag is attached to a GPI-anchor signal which results in surface enyzmatic attachment of the tags to GPI anchors on the cell surface. Both strategies result in high levels of surface expression with a very compact coding sequence. (b) To test surface expression, the 6 different types of surface epitope-tags were co-expressed with enhanced blue fluorescent protein 2 (eBFP2). (c) 293T cells were transfected with these plasmids and the cells stained with anti-tag antibodies and a fluorescent secondary antibody. The cells were then analysed by flow-cytometry and expression of the tag compared with fluorescent signal of eBFP2.

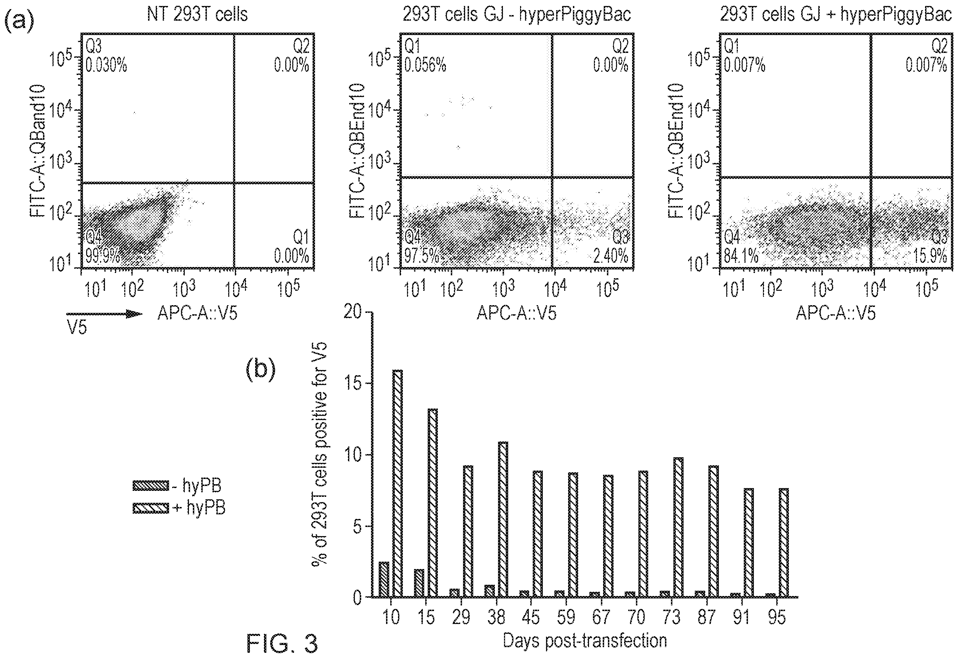

**Figure 3** shows the permanent expression of a helper transgene up to 95 days. (a) 293T cells were co-transfected with a piggyBAC transposase expression plasmid and a lentiviral rev expression plasmid which also co-expressed V5-8 marker gene. Some 293T cells were also transfected with just the rev expression plasmid alone to determine contribution to expression from transient transfection as opposed to permanent insertion. At 10 days, a stable population of V5-8 expression cells can be seen in the transposase co-transfected cells, while expression in the rev expression alone 293T cells is fading. (b) V5-8 expression for both these 293T cell population over time up to 95 days in culture.

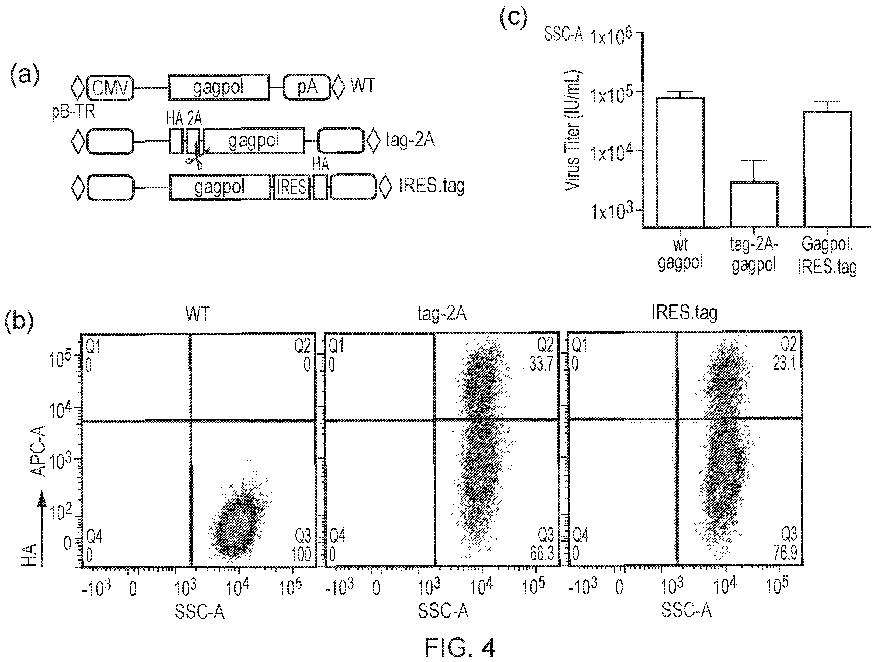

**Figure 4** shows the importance of selecting the correct co-expression mechanism with gagpol. (a) Three lentiviral gagpol expression cassettes were tested. All three were flanked by piggyBAC terminal repeats (pB-TR). Expression was driven by a CMV promoter. The first plasmid (WT) had no epitope tag co-expression. The second plasmid (tag-2A) had an HA-8 tag coded 5' to the gagpol open-reading frame separated by a FMD-disease 2A like peptide coding sequence. In the third plasmid (IRES.tag), and internal ribosome entry sequence (IRES) was inserted 3' to the gagpol frame and the sequence coding for HA-8 followed the IRES. (b) 293T cells transfected with these plasmids were stained with an anti-HA antibody and analysed by flow-cytometry. The tag could be readily detected in both tag-2A and IRES.tag constructs. (c) Lentiviral supernatant was generated by transiently transfecting helper plasmids and transfer vector where the gagpol was supplied by either one of the above three plasmids. Tag-2A construct resulted in a lower virus titer.

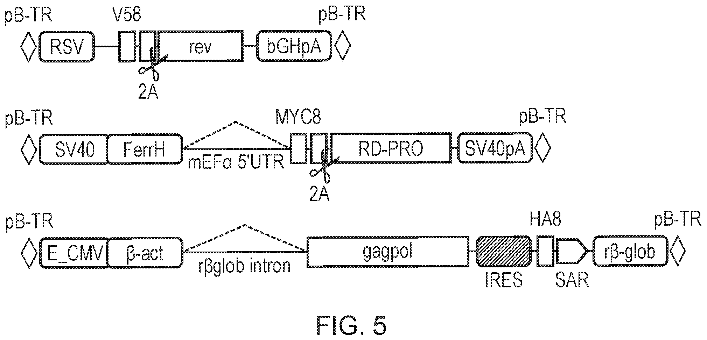

**Figure 5** shows design of optimal lentiviral helper expression cassettes. These cassettes supply rev, RD-PRO and gagpol. Each is flanked by piggyBAC terminal repeats (pB-TR). Promoters, polyadenylation sequences are different to reduce the probability of homologous recombination. Each lentiviral element is co-expressed with a surface epitope marker. Co-expression in the Rev and RD-PRO cassette is achieved with a FMD like 2A peptide. Co-expression in the gagpol cassette is achieved with an IRES. RD-PRO requires an intron for most efficient expression. This is provided by the mouse EFalpha 5' UTR. The gagpol cassette also contains a scaffold attachment region (SAR).

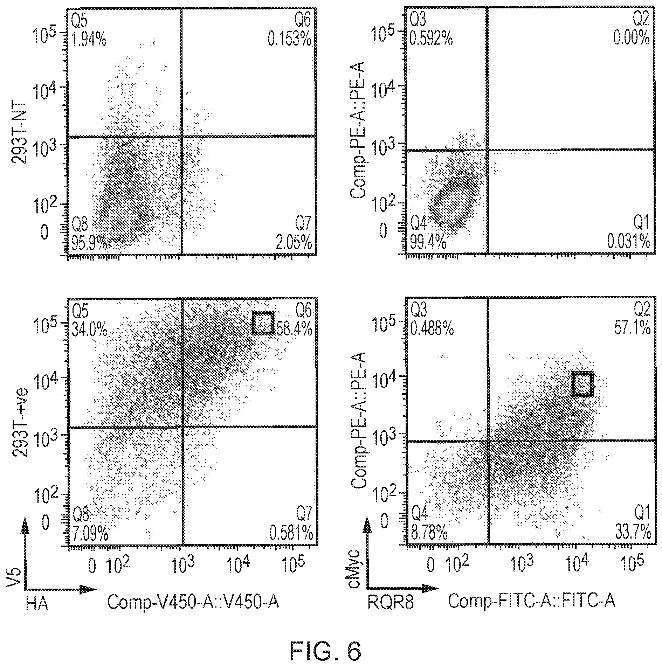

**Figure 6** shows 293T cells stably transposed with lentiviral helper cassettes and a transfer vector. The helper cassettes co-express HA, V5 and MYC tags, while the transfer vector expresses RQR8. All 4 tags can be independently detected and populations of cells which express desired amounts of the different elements can be gated (an example gate is shown) and flow-sorted.

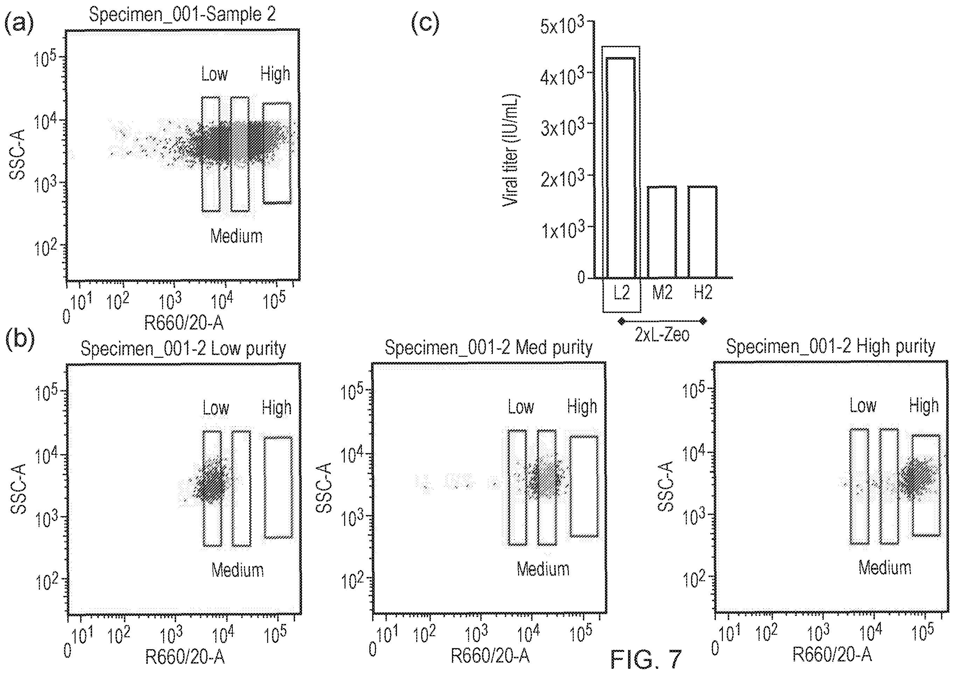

**Figure 7** shows lentiviral vector production from cells transposed with a tagged gagpol expression cassette. (a) 293T cells were transposed. After expression stabilized, 293T cells were sorted into low, medium and high expressing populations using flow-sorting of tag expression; (b) Expression was confirmed immediately after sorting; (c) 293T cells were then transfected with RD114 envelop, transfer vector and REV. Supernatant was harvested and tittered on 293T cells. The lowest expressing cells have the highest titer.

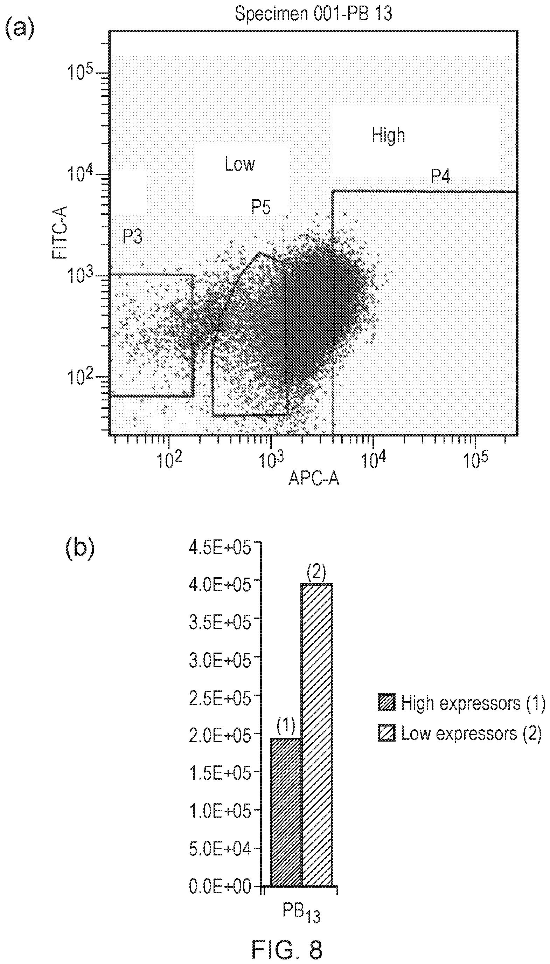

**Figure 8** shows lentiviral vector production from cells transposed with a tagged transfer vector. 293T cells were transposed with a lentiviral transfer vector which also expressed a surface marker. One expression had stabilized, 293T cells were flow sorted into high and low expressing populations using flow-sorting on the marker gene.

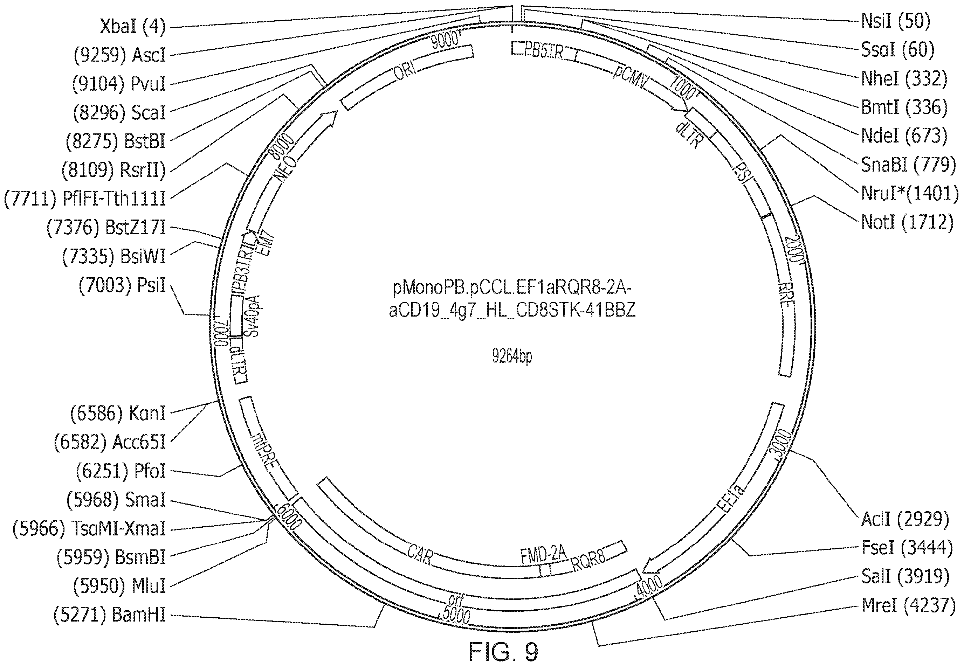

**Figure 9** shows a map of a transposable tagged transfer vector cassette. The 5' end starts with the piggyBAC 5' terminal repeat (5TR). Next, a CMV promoter fused to the 5' lentiviral long-terminal repeat drives expression. Next, the lentiviral packaging signal (PSI) is followed by the lentiviral REV-response element (RRE). Following this is the mature lentiviral expression cassette which has an EF1 alpha promoter driving expression of the transgene which is a fusion between the surface marker gene RQR8 and a CD19 chimeric antigen receptor (CAR). An optimized woodchuck pre-processing element follows (mtPRE) which leads onto the lentiviral 3'LTR truncated to be self-inactivating. Transcription termination is re-enforced by an SV40 polyadenylation signal (SV40pA). Finally, the mammalian cassette ends with the PiggyBAC 3' terminal repeats. The rest of the plasmid contains elements for bacterial propagation.

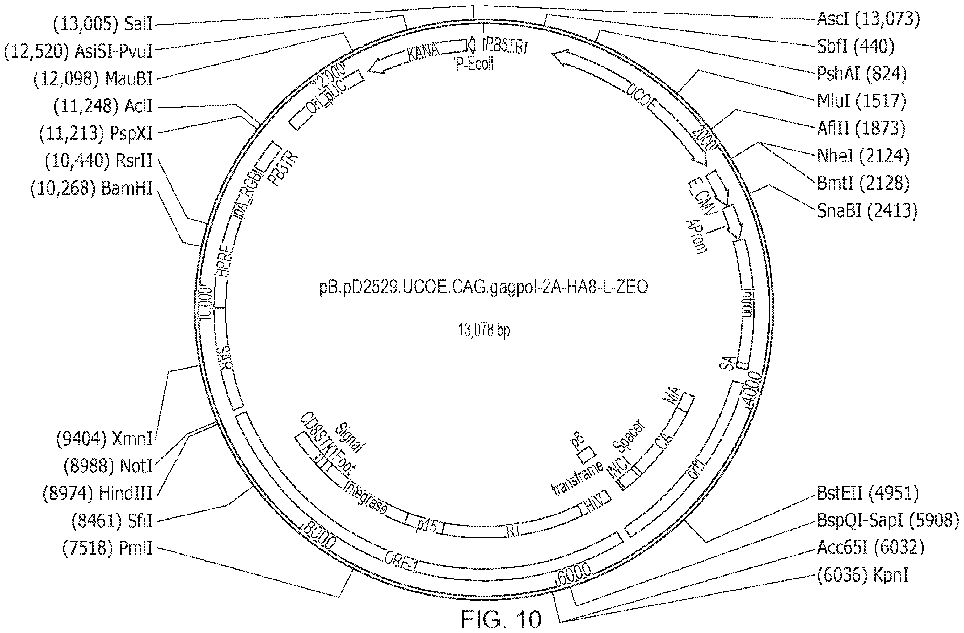

**Figure 10** shows a map of a transposable gagpol expression cassette. The 5' end starts with the piggyBAC 5' terminal repeat (5TR). This is followed by a ubiquitin opening element (UCOE). This is followed by the CMV promoter and an intron. Next, is the staggered lentiviral gagpol open reading frame. Cloned in frame with the end of this is a 2A peptide and an epitope tag on a CD8 stalk (CD8STK). Next is a scaffold attachment region (SAR), a pre-processing element (PRE) and the polyadenylation signal from the rabbit beta-globin gene (RGBpA). The mammalian cassette ends with the piggyBAC 3' terminal repeat (3TR). The rest of the plasmid contains elements for bacterial propagation.

### DETAILED DESCRIPTION OF THE INVENTION

The present invention provides a nucleic acid construct comprising
(i) a first nucleic acid sequence which encodes a retroviral protein; and
(ii) a second nucleic acid sequence which encodes a detectable marker which is a cell surface protein comprising an extracellular domain and a membrane targeting domain.

The nucleic acid construct has the structure: A-X-B; in which A is the first nucleic acid sequence, B is the second nucleic acid sequence and X is a co-expression sequence.

The nucleic acid sequence may be an RNA or DNA sequence or a variant thereof.

### RETROVIRUS

The concept of using viral vectors for gene therapy is well known (Verma and Somia (1997) Nature 389:239-242). As used herein the term "retroviral vector", when referring to a retroviral vector system also includes a retroviral vector particle capable of transducing a recipient cell with a nucleotide of interest (NOI).

A retroviral vector particle includes the following components: a vector genome, which may contain one or more NOIs, a nucleocapsid encapsidating the nucleic acid, and a membrane surrounding the nucleocapsid.

The term "nucleocapsid" refers to at least the group specific viral core proteins (gag) and the viral polymerase (pol) of a retrovirus genome. These proteins encapsidate the packagable sequences and are themselves further surrounded by a membrane containing an envelope glycoprotein.

The term "vector genome" refers to both to the RNA construct present in the retroviral vector particle and the integrated DNA construct. The term also embraces a separate or isolated DNA construct capable of encoding such an RNA genome. A retroviral genome should comprise at least one component part derivable from a retrovirus. The term "derivable" is used in its normal sense as meaning a nucleotide sequence or a part thereof which need not necessarily be obtained from a virus such as a lentivirus but instead could be derived therefrom. By way of example, the sequence may be prepared synthetically or by use of recombinant DNA techniques.

There are many retroviruses. For the present application, the term "retrovirus" includes, but is not limited to: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other retroviridiae including lentiviruses.

A detailed list of retroviruses may be found in Coffin et al ("Retroviruses" 1997 Cold Spring Harbour Laboratory Press Eds: JM Coffin, SM Hughes, HE Varmus pp 758-763).

In a preferred embodiment, the retroviral vector is derivable from a lentivirus.

Lentiviral vectors are major tools for gene delivery, providing efficient transduction of a wide variety cell types such as hematopoietic stem cells, neurons and endothelial cells. The advantages of lentiviral vectors over other systems are the ability to infect both dividing and non-dividing cells *in vivo* and *in vitro* and their greater packaging capacity that enables the expression of larger RNA transcripts.

The lentivirus group can be split into "primate" and "non-primate". Examples of primate lentiviruses include the human immunodeficiency virus (HIV), the causative agent of human acquired immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

Details on the genomic structure of some lentiviruses may be found in the art. By way of example, details on HIV and EIAV may be found from the NCBI Genbank database (i.e. Genome Accession Nos. AF033819 and AF033820 respectively). Details of HIV variants may also be found at http://hiv-web.lanl.gov. Details of EIAV variants may be found through http://www.ncbi.nlm.nih.gov.

Lentiviruses have three main genes coding for the viral proteins in the order: 5'-gag-pol-env-3'. There are two regulatory genes, tat and rev. There are additional accessory genes depending on the virus (e.g., for HIV-1: vif, vpr, vpu, nef) whose products are involved in regulation of synthesis and processing viral RNA and other replicative functions. The Long terminal repeat (LTR) is about 600nt long, of which the U3 region is 450, the R sequence 100 and the U5 region some 70 nt long.

Viral proteins involved in early stages of replication include Reverse Transcriptase and Integrase. Reverse Transcriptase is the virally encoded RNA-dependent DNA polymerase. The enzyme uses the viral RNA genome as a template for the synthesis of a complementary DNA copy. Reverse transcriptase also has RNaseH activity for destruction of the RNA-template. Integrase binds both the viral cDNA generated by reverse transcriptase and the host DNA. Integrase processes the LTR before inserting the viral genome into the host DNA. Tat acts as a trans-activator during transcription to enhance initiation and elongation. The Rev responsive element acts post-transcriptionally, regulating mRNA splicing and transport to the cytoplasm.

### RETROVIRAL PROTEIN

The retroviral protein may be Gag, Pol, Env or Rev.

Group-specific antigen (gag) proteins are major components of the viral capsid, which are about 2000-4000 copies per virion. Pol proteins are responsible for synthesis of viral DNA and integration into host DNA after infection. Env proteins play a role in association and entry of virions into the host cell. A functional copy of an *env* gene is what makes retroviruses distinct from retroelements. The ability of the retrovirus to bind to its target host cell using specific cell-surface receptors is given by the surface component (SU) of the Env protein, while the ability of the retrovirus to enter the cell via membrane fusion is imparted by the membrane-anchored trans-membrane component (TM). Thus the Env protein is what enables the retrovirus to be infectious.

The retroviral protein may be gagpol. The nucleic acid sequence which encodes gagpol may comprise the nucleic acid sequence shown as SEQ ID NO: 9. The nucleic acid sequence which encodes gagpol may comprise a variant of SEQ ID NO: 9 which shares at least 70%, at least 75%, at least 80%, at least 85%, at least 90%, at least 95% or at least 99% sequence identity to SEQ ID NO: 9 and encodes functional Gag and Pol proteins.
SEQ ID NO: 9 - ***Gag*.Pol** - DNA sequence

The retroviral env protein may be the RD114 SU or TM protein or the RDPRO SU or TM protein. In one embodiment, the env protein is VSV-G (glycoprotein G of the Vesicular stomatitis virus (VSV)). VSV-G is a commonly used env protein for lentiviral pseudotyping as it is capable of transducing all cell types.

The retroviral env protein may comprise the sequence shown as SEQ ID NO: 10. The retroviral env protein may comprise a variant of SEQ ID NO: 10 which retains ability to provide a functional env protein. The variant may share at least 70%, at least 75%, at least 80%, at least 85%, at least 90%, at least 95% or at least 99% sequence identity to SEQ ID NO: 10.
SEQ ID NO: 10 - VSVG env amino acid sequence

Suitably, 'Retroviral envelope protein' refers to the SU and/or TM proteins, as described above in relation to lentiviruses.

In one embodiment the packaging or producer cell is used to produce a lentivirus vector and retroviral protein may be Rev. Rev is specifically expressed by lentivirus and acts post-transcriptionally, regulating mRNA splicing and transport to the cytoplasm.

### DETECTABLE MARKER

The detectable marker is a cell surface protein. The cell surface protein may be any cell surface protein which is not natively expressed on the surface of the cell type which is used as the packaging cell or producer cell.

The cell surface protein comprises a membrane targeting domain and an extracellular domain. When expressed at the cell surface at least one domain of the cell surface protein is exoplasmic (i.e. on the exterior of the cell). This domain of the cell surface protein is therefore accessible for antibody binding.

In one embodiment, the detectable marker is a cell surface protein where the expression level can be determined at the single cell level. In particular, the detectable marker may be detected without disrupting the cell. Because the expression level of the detectable marker can be determined at the single cell level, the co-expression of the detectable marker with a retroviral protein means that the expression level of the retroviral protein within a packaging cell can also be determined.

This is in contrast to the selectable antibiotic resistance markers previously used to identify packaging cells expressing a retroviral protein. In particular, although the use of selectable antibiotic resistance markers enables cells which express the resistance marker - and therefore the retroviral protein - to be selected, it is difficult to determine the relative expression obtained from each individual integration event and thus the level of expression of each retroviral protein in a particular cell.

Accordingly, the detectable marker of the present invention is not an antibiotic resistance marker.

For example, the expression level of the detectable marker may be determined using flow cytometry. Methods for using techniques such as flow cytometry to determine the expression levels of proteins are well known in the art. As such, the detectable marker may be a cell surface protein which can be detected using flow cytometry by using a reagent(s) which allows expression of the cell surface protein to be determined. For example the reagent may be an antibody, for example a labelled antibody such as a fluorescently labelled antibody which specifically binds the cell surface protein. The detectable marker may be a fluorescent protein which is inherently detectable by flow cytometry due to its fluorescent characteristics.

In one embodiment the cell surface protein is less than 200 amino acids in length. The use of a cell surface protein of less than 200 amino acids is advantageous because the smaller nucleic acid sequences which encode such proteins are easier to transfect and stably integrate into cells.

### Membrane targeting domain

A 'membrane targeting domain' is an entity which preferentially localises to the membrane and therefore anchors the cell surface protein to the membrane of, for example, a packaging cell.

A transmembrane domain is a hydrophobic alpha helix which spans a cell membrane and typically found in transmembrane proteins. It may comprise a hydrophobic alpha helix. The transmembrane domain may be derived from CD8, CD28 or human IgG.

The transmembrane domain may be derived from any type I transmembrane protein. The transmembrane domain may be a synthetic sequence predicted to form a hydrophobic helix.

The transmembrane domain may comprise the sequence shown as SEQ ID NO: 12.
SEQ ID NO: 12 (CD28 transmembrane domain)
FVVVLVVVGGVLACYSLLVTVAFIIFWV

The membrane targeting domain may be a transmembrane domain and an endodomain, which orientates to the interior of the packaging cell. The endodomain may comprise polar residues which anchor the cell surface protein to the membrane.

The membrane targeting domain may be a GPI anchor.

GPI anchoring is a post-translational modification which occurs in the endoplasmic reticulum. Preassembled GPI anchor precursors are transferred to proteins bearing a C-terminal GPI signal sequence (see Kinoshita et al.; J Biochem; 122, 251-257 (1997)). During processing, the GPI anchor replaces the GPI signal sequence and is linked to the target protein via an amide bond. The GPI anchor targets the mature protein to the membrane.

The present tagging protein may comprise a GPI signal sequence. For example, the tagging protein may comprise a sequence shown as SEQ ID NO: 13.
SEQ ID NO : 13
ASSNISGGIFLFFVANAIIHLFCFS

### Extracellular domain

The extracellular domain is the part of the cell surface protein that exoplasmic (i.e. on the exterior of the cell) when the cell surface protein is expressed at the cell surface.

The extracellular domain may be any polypeptide sequence which comprises an epitope that can be specifically bound by an antibody.

In one embodiment the extracellular domain is less than 200 amino acids in length.

The extracellular domain may comprise a HA, V5, RQR-8 or MYC tag.

Human influenza hemagglutinin (HA) is a surface glycoprotein required for the infectivity of the human virus. The HA tag is derived from the HA-molecule corresponding to amino acids 98-106. It has been extensively used as a general epitope tag in expression vectors. The HA tag may comprise the sequence shown as SEQ ID NO: 14.
YPYDVPDYA (SEQ ID NO: 14)

The V5 epitope tag (V5) is derived from a small epitope (Pk) present on the P and V proteins of the paramyxovirus of simian virus 5 (SV5). The V5 tag may comprise the sequence shown as SEQ ID NO: 15 or 16.
GKPIPNPLLGLDST (SEQ ID NO: 15)
IPNPLLGLD (SEQ ID NO: 16)

A myc tag (MYC) is a polypeptide protein tag derived from the c-myc gene product. The MYC tag may comprise the sequence shown as SEQ ID NO: 17.
EQKLISEEDL (SEQ ID NO: 17)

The extracellular domain may comprise a domain which comprises a rituximab-binding epitope (R epitope) and/or a Qbend10 epitope (Q epitope). A rituximab-binding epitope refers to an epitope which specifically binds rituximab. For example, the rituximab-binding epitope may be based on the CD20 B-cell antigen.

The Rituximab-binding epitope sequence from CD20 is CEPANPSEKNSPSTQYC (SEQ ID NO: 18)
The CliniMACS CD34 selection system utilises the QBEnd10 monoclonal antibody to achieve cellular selection. The present inventors have previously mapped the QBEnd10-binding epitope from within the CD34 antigen (see WO 2013/153391) and determined it to have the amino acid sequence shown as SEQ ID NO: 19.
ELPTQGTFSNVSTNVS (SEQ ID NO: 19).

The binding domain of the cell surface protein may comprise a QBEnd10-binding epitope having the amino acid sequence shown as SEQ ID NO: 19 or a variant thereof which retains QBEnd10-binding activity.

The extracellular domain may comprise a binding domain which comprises or consists of 136 amino acid sequence shown as SEQ ID NO: 20.

The cell surface marker protein may comprise or consist of a sequence shown as SEQ ID NO: 21 to 26.

The cell surface protein may comprise a variant of an extracellular domain or a cell surface marker protein as described herein. For example, the extracellular domain may be based on a sequence provided herein but comprises one or more amino acid mutations, such as amino acid insertions, substitutions or deletions, provided that the epitope retains binding activity of the entity or epitope on which it is based. In particular, the sequence may be truncated at one or both terminal ends by, for example, one or two amino acids.

Deliberate amino acid substitutions may be made on the basis of similarity in polarity, charge, solubility, hydrophobicity, hydrophilicity, and/or the amphipathic nature of the residues as long as binding activity of the epitope is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and in the same line in the third column may be substituted for each other.

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The binding domain may, for example, contain 5 or fewer, 4 or fewer, 3 or fewer, 2 or fewer or 1 amino acid mutation(s) compared to a binding domain sequence as shown herein.

The extracellular domain may consist essentially of a domain sequence as shown herein or a variant thereof which retains the ability to bind an antibody which binds to the domain on which it is based. The extracellular domain may consist of a sequence as shown herein or a variant thereof which retains the ability to bind an antibody which binds to the domain on which it is based.

The cell surface protein may comprise the sequence shown as SEQ ID NO: 14 to 26 or a variant thereof having 80% identity thereto which retains the ability to be expressed at the cell surface and recognised by reagent, for example, an antibody which binds to one of SEQ ID NO: 14 to 26.

The cell surface protein may comprise the sequence shown as SEQ ID NO: 14 to 26 or a variant thereof having 85% identity thereto which retains the ability to be expressed at the cell surface and recognised by an antibody or a ligand which binds to one of SEQ ID NO: 14 to 26

The cell surface protein may comprise the sequence shown as SEQ ID NO: 14 to 26 or a variant thereof having 90% identity thereto which retains the ability to be expressed at the cell surface and recognised by an antibody or a ligand which binds to one of SEQ ID NO: 14 to 26

The cell surface protein may comprise the sequence shown as SEQ ID NO: 14 to 26 or a variant thereof having 95% identity thereto which retains the ability to be expressed at the cell surface and recognised by an antibody or a ligand which binds to one of SEQ ID NO: 14 to 26

The cell surface protein may comprise the sequence shown as SEQ ID NO: 14 to 26 or a variant thereof having 99% identity thereto which retains the ability to be expressed at the cell surface and recognised by an antibody or a ligand which binds to one of SEQ ID NO: 14 to 26

### Signal sequence

The cell surface protein may also comprise a signal sequence so that when the protein is expressed inside a cell the nascent protein is directed to the endoplasmic reticulum (ER).

The core of the signal peptide may contain a long stretch of hydrophobic amino acids that has a tendency to form a single alpha-helix. The signal peptide may begin with a short positively charged stretch of amino acids, which helps to enforce proper topology of the polypeptide during translocation. At the end of the signal peptide there is typically a stretch of amino acids that is recognized and cleaved by signal peptidase. Signal peptidase may cleave either during or after completion of translocation to generate a free signal peptide and a mature protein. The free signal peptides are then digested by specific proteases.

The signal peptide may be at the amino terminus of the molecule.

The signal peptide may comprise the SEQ ID NO: 1 or 2 or a variant thereof having 5, 4, 3, 2 or 1 amino acid mutations (insertions, substitutions or additions) provided that the signal peptide still functions to cause secretion of the bi-specific molecule.
METDTLLLWVLLLWVPGSTG (SEQ ID NO: 1)
MGTSLLCWMALCLLGADHADG (SEQ ID NO: 2)

The signal peptides of SEQ ID NO: 1 and 2 are compact and highly efficient. They are predicted to give about 95% cleavage after the terminal glycine, giving efficient removal by signal peptidase.

The cell surface marker protein as described herein may lack a signal sequence. For example, the cell surface marker shown as SEQ ID NO: 21-26 may not include the sequence annotated of the signal sequence. In particular, the cell surface marker protein will not include the signal sequence when the protein is present at the cell surface.

### CO-EXPRESSION SEQUENCE

The retroviral protein and the detectable marker are transcribed as a nucleic acid which comprises a nucleic acid sequence which encodes the retroviral protein, a co-expression sequence and a nucleic acid sequence which encodes the detectable marker, wherein the co-expression sequence enables the retroviral protein and the detectable marker to be expressed as separate polypeptides.

The co-expression sequence may be a 2A self-cleaving peptide from an aphtho- or a cardiovirus, a 2A-like peptide, an internal ribosome entry site, a self-cleaving peptide, a furin cleavage site or a Tobacco Etch Virus cleavage site.

An internal ribosome entry site (IRES) is a nucleotide sequence that allows for translation initiation in the middle of a messenger RNA (mRNA).

The co-expression sequence may be a sequence encoding a cleavage site positioned between nucleic acid sequences which encode the retroviral protein and detectable marker, such that the retroviral protein and detectable marker can be expressed as separate entities.

The cleavage site may be any sequence which enables the polypeptide comprising the retroviral protein and detectable marker to become separated.

The term "cleavage" is used herein for convenience, but the cleavage site may cause the retroviral protein and detectable marker 'to separate into individual entities by a mechanism other than classical cleavage. For example, for the Foot-and-Mouth disease virus (FMDV) 2A self-cleaving peptide (see below), various models have been proposed for to account for the "cleavage" activity: proteolysis by a host-cell proteinase, autoproteolysis or a translational effect (Donnelly et al (2001) J. Gen. Virol. 82:1027-1041). The exact mechanism of such "cleavage" is not important for the purposes of the present invention, as long as the cleavage site, when positioned between nucleic acid sequences which encode retroviral protein and detectable marker, causes the retroviral protein and detectable marker to be expressed as separate entities.

The cleavage site may be a furin cleavage site.

Furin is an enzyme which belongs to the subtilisin-like proprotein convertase family. The members of this family are proprotein convertases that process latent precursor proteins into their biologically active products. Furin is a calcium-dependent serine endoprotease that can efficiently cleave precursor proteins at their paired basic amino acid processing sites. Examples of furin substrates include proparathyroid hormone, transforming growth factor beta 1 precursor, proalbumin, pro-beta-secretase, membrane type-1 matrix metalloproteinase, beta subunit of pro-nerve growth factor and von Willebrand factor. Furin cleaves proteins just downstream of a basic amino acid target sequence (canonically, Arg-X-(Arg/Lys)-Arg') (SEQ ID NO: 3) and is enriched in the Golgi apparatus.

The cleavage site may be a Tobacco Etch Virus (TEV) cleavage site.

TEV protease is a highly sequence-specific cysteine protease which is chymotrypsin-like proteases. It is very specific for its target cleavage site and is therefore frequently used for the controlled cleavage of fusion proteins both *in vitro* and *in vivo.* The consensus TEV cleavage site is ENLYFQ\S (SEQ ID NO: 4) (where '\' denotes the cleaved peptide bond). Mammalian cells, such as human cells, do not express TEV protease. Thus in embodiments in which the present nucleic acid construct comprises a TEV cleavage site and is expressed in a mammalian cell - exogenous TEV protease must also expressed in the mammalian cell.

The cleavage site may encode a self-cleaving peptide.
A 'self-cleaving peptide' refers to a peptide which functions such that when the polypeptide comprising the retroviral protein and detectable marker and the self-cleaving peptide is produced, it is immediately "cleaved" or separated into distinct and a discrete retroviral protein and a detectable marker without the need for any external cleavage activity.

The self-cleaving peptide may be a 2A self-cleaving peptide from an aphtho- or a cardiovirus. The primary 2A/2B cleavage of the aptho- and cardioviruses is mediated by 2A "cleaving" at its own C-terminus. In apthoviruses, such as foot-and-mouth disease viruses (FMDV) and equine rhinitis A virus, the 2A region is a short section of about 18 amino acids, which, together with the N-terminal residue of protein 2B (a conserved proline residue) represents an autonomous element capable of mediating "cleavage" at its own C-terminus.

The C-terminal 19 amino acids of the longer cardiovirus protein, together with the N-terminal proline of 2B mediate "cleavage" with an efficiency approximately equal to the apthovirus FMDV 2a sequence. Cardioviruses include encephalomyocarditis virus (EMCV) and Theiler's murine encephalitis virus (TMEV).

Mutational analysis of EMCV and FMDV 2A has revealed that the motif DxExNPGP (SEQ ID NO: 5) is intimately involved in "cleavage" activity (Donelly et al (2001) as above).

The cleavage site of the present invention may comprise the amino acid sequence: Dx₁Ex₂NPGP, where x₁ and x₂ are any amino acid (SEQ ID NO: 5). Suitably, X₁ may be selected from the following group: I, V, M and S. X₂ may be selected from the following group: T, M, S, L, E, Q and F (as shown in SEQ ID NO: 8).

For example, the cleavage site may comprise one of the amino acid sequences shown in Table 1.

**Table 1**

| Motif | Present in: |
|---|---|
| DIETNPGP (SEQ ID NO: 6) | Picornaviruses EMCB, EMCD, EMCPV21 |
| DVETNPGP (SEQ ID NO: 7) | Picornaviruses MENGO and TMEBEAN; Insect virus DCV, ABPV |
| DVEMNPGP (SEQ ID NO: 27) | Picornaviruses TMEGD7 and TMEBEAN |
| DVESNPGP (SEQ ID NO: 28) | Picornaviruses FMDA10, FMDA12, FMDC1, FMD01K, FMDSAT3, FMDVSAT2, ERAV; Insect virus CrPV |
| DMESNPGP (SEQ ID NO: 29) | Picornavirus FMDV01G |
| DVELNPGP (SEQ ID NO: 30) | Picornavirus ERBV; Porcine rotavirus |
| DVEENPGP (SEQ ID NO: 31) | Picornavirus PTV-1; Insect virus TaV; Trypanosoma TSR1 |
| DIELNPGP (SEQ ID NO: 32) | Bovine Rotavirus, human rotavirus |
| DIEQNPGP (SEQ ID NO: 33) | Trypanosoma AP endonuclease |
| DSEFNPGP (SEQ ID NO: 34) | Bacterial sequence *T. maritima* |

The cleavage site, based on a 2A sequence may be, for example 15-22 amino acids in length. The sequence may comprise the C-terminus of a 2A protein, followed by a proline residue (which corresponds to the N-terminal proline of 2B).

Mutational studies have also shown that, in addition to the naturally occurring 2A sequences, some variants are also active. The cleavage site may correspond to a variant sequence from a naturally occurring 2A polypeptide, have one, two or three amino acid substitutions, which retains the capacity to induce the "cleavage" of a polyprotein sequence into two or more separate proteins.

The cleavage sequence may be selected from the following which have all been shown to be active to a certain extent (Donnelly *et al* (2001) as above):
LLNFDLLKLAGDVESNPGP (SEQ ID NO: 35)
LLNFDLLKLAGDVQSNPGP (SEQ ID NO: 36)
LLNFDLLKLAGDVEINPGP (SEQ ID NO: 37)
LLNFDLLKLAGDVEFNPGP (SEQ ID NO: 38)
LLNFDLLKLAGDVESHPGP (SEQ ID NO: 39)
LLNFDLLKLAGDVESEPGP (SEQ ID NO: 40)
LLNFDLLKLAGDVESQPGP (SEQ ID NO: 41)
LLNFDLLKLAGDVESNPGG (SEQ ID NO: 42)

Based on the sequence of the DxExNPGP "a motif, "2A-like" sequences have been found in picornaviruses other than aptho- or cardioviruses, 'picornavirus-like' insect viruses, type C rotaviruses and repeated sequences within *Trypanosoma spp* and a bacterial sequence (Donnelly et al (2001) as above). The cleavage site may comprise one of these 2A-like sequences, such as:
YHADYYKQRLIHDVEMNPGP (SEQ ID NO: 43)
HYAGYFADLLIHDIETNPGP (SEQ ID NO: 44)
QCTNYALLKLAGDVESNPGP (SEQ ID NO: 45)
ATNFSLLKQAGDVEENPGP (SEQ ID NO: 46)
AARQMLLLLSGDVETNPGP (SEQ ID NO: 47)
RAEGRGSLLTCGDVEENPGP (SEQ ID NO: 48)
TRAEIEDELIRAGIESNPGP (SEQ ID NO: 49)
TRAEIEDELIRADIESNPGP (SEQ ID NO: 50)
AKFQIDKILISGDVELNPGP (SEQ ID NO: 51)
SSIIRTKMLVSGDVEENPGP (SEQ ID NO: 52)
CDAQRQKLLLSGDIEQNPGP (SEQ ID NO: 53)
YPIDFGGFLVKADSEFNPGP (SEQ ID NO: 54)

The cleavage site may comprise the 2A-like sequence shown as SEQ ID NO: 55 (RAEGRGSLLTCGDVEENPGP).

It has been shown that including an N-terminal "extension" of between 5 and 39 amino acids can increase activity (Donnelly et al (2001) as above). In particular, the cleavage sequence may comprise one of the following sequences or a variant thereof having, for example, up to 5 amino acid changes which retains cleavage site activity:
VTELLYRMKRAETYCPRPLAIHPTEARHKQKIVAPVKQTLNFDLLKLAGDVESNPGP (SEQ ID NO: 56)
LLAIHPTEARHKQKIVAPVKQTLNFDLLKLAGDVESNPGP (SEQ ID NO: 57)
EARHKQKIVAPVKQTLNFDLLKLAGDVESNPGP (SEQ ID NO: 58)
APVKQTLNFDLLKLAGDVESNPGP (SEQ ID NO: 59)

### TRANSPOSON ELEMENT

Various transposon elements are well known in the art. Any suitable transposon may be used in the present invention.

DNA transposons are genetic elements that can mobilize from one location to another in the host genome. Their use in engineering mammalian cells was initially hampered by the lack of active transposons in mammals. Over a decade ago, the first active DNA transposon in mammals, Sleeping Beauty, was reconstructed from fossilized transposon sequences found in the salmonid genome. This discovery made transposon mediated genetic modification of mammalian cells possible. This method is much more efficient at generating cell lines with modified genomes than simple transfection.

Since the generation of the Sleeping Beauty transposon, a number of transposons from different families have been reported to show active transposition in mammalian cells (Skipper et al, J of Biomed Sci 2013, 20:92). Currently, along with Sleeping Beauty, the piggyBac (PB) transposon isolated from cabbage looper moth *Trichoplusia ni* is most promising because of a variety of unique characteristics, namely exhibiting the most efficient transposition in mammalian cells, the ability of the transposase to form functional protein fusions, large cargo capacity, and traceless excision. More importantly, PB has been found capable of mediating stable integration of up to 4 independent transposons simultaneously in human cells following a single transfection. A more active form of piggyback has been described (Yusa et al, PNAS, 2011, vol. 108, no.4).

In one embodiment of the invention, the transposon element or sequence comprises (or consists of) a piggyBac transposon. During transposition, the PB transposase recognizes transposon-specific inverted terminal repeat sequences (ITRs) located on both ends of the transposon vector and efficiently moves the contents from the original sites and integrates them into TTAA chromosomal sites.

Unique features of PiggyBac transposons are that there is relatively no cargo limit as it can carry a transgene of up to 14kbp and it is also reversible: genomes containing an inserted PiggyBac vector can be transiently re-transfected with the PB tranposase expression vector and the transposase will remove the transposons from the genome, footprint-free.

In one embodiment, the nucleic acid construct may comprise a PiggyBAC 5' terminal repeat and a PiggyBAC 3' terminal repeat such that the construct can insert into the host genome. The PiggyBAC 5' and 3' terminal repeats may flank the sequence which is to be inserted into the host genome.

The PiggyBAC 5' Terminal Repeat may comprise or consist of a variant which shares at least 80%, at least 85%, at least 90%, at least 95%, or at least 99% sequence identity with SEQ ID NO: 60 and retains the ability to recruit an RdRp. The PiggyBAC 5' Terminal Repeat may comprise or consist of SEQ ID NO: 60.

The PiggyBAC 5' Terminal Repeat may comprise or consist of a truncated version of SEQ ID NO: 60 or truncated version of a variant of SEQ ID NO: 60 which retains ability to facilitate insertion into the host genome.

The PiggyBAC 3' Terminal Repeat may comprise or consist of a variant which shares at least 80%, at least 85%, at least 90%, at least 95%, or at least 99% sequence identity with SEQ ID NO: 61 and retains the ability to recruit an RdRp. The PiggyBAC 3' Terminal Repeat may comprise or consist of SEQ ID NO: 61.

The PiggyBAC 3' Terminal Repeat may comprise or consist of a truncated version of SEQ ID NO: 61 or truncated version of a variant of SEQ ID NO: 61 which retains ability to facilitate insertion into the host genome.

### PLASMID

In one aspect the present invention provides a plasmid comprising a nucleic acid construct of the present invention.

The term plasmid covers any DNA transcription unit comprising a nucleic acid construct according to the invention and the elements necessary for its *in vivo* expression in a desired cell; and, in this regard, it is noted that a supercoiled or non-supercoiled, circular plasmid, as well as a linear form, are intended to be within the scope of the invention.

A plasmid may comprise the nucleic acid construct according to the invention, operably linked to a promoter or under the control of a promoter or dependent upon a promoter. In general, it is advantageous to employ a strong promoter functional in eukaryotic cells. The strong promoter may be, but not limited to, the immediate early cytomegalovirus promoter (CMV-IE) of human or murine origin, or optionally having another origin such as the rat or guinea pig.

In more general terms, the promoter has either a viral, or a cellular origin. A strong viral promoter other than CMV-IE that may be usefully employed in the practice of the invention is the early/late promoter of the SV40 virus or the LTR promoter of the Rous sarcoma virus. A strong cellular promoter that may be usefully employed in the practice of the invention is the promoter of a gene of the cytoskeleton, such as e.g. the desmin promoter (Kwissa et al., 2000), or the actin promoter (Miyazaki et al., 1989).

As to the polyadenylation signal (polyA) for the plasmids use can be made of the poly(A) signal of the bovine growth hormone (bGH) gene (see U.S. 5,122,458), or the poly(A) signal of the rabbit β-globin gene or the poly(A) signal of the SV40 virus.

### KIT

The present invention further provides a kit comprising a plurality of nucleic acid constructs of the invention.

In one embodiment the kit may comprise:
(i) a first nucleic acid construct comprising a *gag-pol* sequence and a sequence encoding a first detectable marker;
(ii) a second nucleic acid construct comprising an *env* sequence and a sequence encoding a second detectable marker;
in which the first and second detectable markers are cell surface proteins comprising an extracellular domain and a membrane targeting domain and are different to each other.

The kit may further comprise:
(iii) a third nucleic acid construct comprising a *rev* sequence and a sequence encoding a third detectable marker which is a cell surface protein comprising an extracellular domain and a membrane targeting domain;
in which the first, second and third and detectable markers are all different to each other.

The kit may also comprise:
(iv) a fourth nucleic acid construct comprising a retroviral transfer vector and a sequence encoding a fourth detectable marker which is a cell surface protein comprising an extracellular domain and a membrane targeting domain;
in which the first, second, third (if present) and fourth detectable markers are all different to each other.

In another aspect described herein is a kit comprising a plurality of plasmids according to the invention.

### PACKAGING CELL AND PRODUCER CELL

As used herein, the term "packaging cell" refers to a cell which contains those elements necessary for production of infectious recombinant virus which are lacking in the RNA genome. Such packaging cells are capable of expressing viral structural proteins (such as gag-pol and env, which may be codon optimised) but they do not contain a packaging signal. The term "packaging signal" which is referred to interchangeably as "packaging sequence" or "psi" is used in reference to the non-coding, cis-acting sequence required for encapsidation of retroviral RNA strands during viral particle formation. In HIV-1, this sequence has been mapped to loci extending from upstream of the major splice donor site (SD) to at least the gag start codon.

As used herein, the term "producer cell" refers to a cell which contains all the elements necessary for production of retroviral vector particles.

The producer cells/packaging cells of the present invention may be any suitable cell type. Producer cells are generally mammalian cells but can be, for example, insect cells.

By using producer/packaging cell lines, it is possible to propagate and isolate quantities of retroviral vector particles (e.g. to prepare suitable titres of the retroviral vector particles) for subsequent transduction of a site of interest.

The packaging cell lines are useful for providing the gene products necessary to encapsidate and provide a membrane protein for a high titre vector particle production. The packaging cell may be a cell cultured *in vitro* such as a tissue culture cell line. Suitable cell lines include but are not limited to mammalian cells such as murine fibroblast derived cell lines or human cell lines. The packaging cell line may be a human cell line, such as for example: HEK293, 293-T, TE671, HT1080.

There are two common procedures for generating producer cells. In one, the sequences encoding retroviral Gag, Pol and Env proteins are introduced into the cell and stably integrated into the cell genome; a stable cell line is produced which is referred to as the packaging cell line. As used herein, the term "stably integrated" means that the foreign genes become integrated into the cell's genome. The packaging cell line produces the proteins required for packaging retroviral RNA but it cannot bring about encapsidation due to the lack of a psi region.

However, when a vector genome (having a psi region) is introduced into the packaging cell line, the helper proteins can package the psi-positive recombinant vector RNA to produce the recombinant virus stock. This can be used to transduce recipient cells. The recombinant virus whose genome lacks all genes required to make viral proteins can infect only once and cannot propagate. Hence, a nucleic acid sequence can be introduced into a host cell genome without the generation of potentially harmful retrovirus.

The second approach is to introduce the three different DNA sequences that are required to produce a retroviral vector particle (i.e. the env coding sequences, the gag-pol coding sequence and the defective retroviral genome containing one or more NOIs) into the cell at the same time by transient transfection and the procedure is referred to as transient triple transfection. WO 94/29438 describes the production of producer cells *in vitro* using this multiple DNA transient transfection method. WO 97/27310 describes a set of DNA sequences for creating retroviral producer cells either in vivo or in vitro for re-implantation.

The components of the viral system which are required to complement the vector genome may be present on one or more "producer plasmids" for transfecting into cells.

The present invention provides a packaging cell comprising a nucleic acid construct according to the invention, and which cell expresses at least one detectable marker which is a cell surface protein comprising an extracellular domain and a membrane targeting domain.

In one embodiment, the packaging cell comprises:
(i) a first nucleic acid construct comprising a *gag-pol* sequence and a sequence encoding a first detectable marker;
(ii) a second nucleic acid construct comprising an *env* sequence and a sequence encoding a second detectable marker;
which co-expresses the first and second detectable markers at the cell surface.

The packaging cell may further comprise:
(iii) a third nucleic acid construct comprising a *rev* sequence and a sequence encoding a third detectable marker;
which co-expresses the first, second and third detectable markers at the cell surface.

The invention further provides a producer cell which comprises:
(i) a first nucleic acid construct comprising a *gag-pol* sequence and a sequence encoding a first detectable marker;
(ii) a second nucleic acid construct comprising an *env* sequence and a sequence encoding a second detectable marker;
(iii) optionally a third nucleic acid construct comprising a *rev* sequence and a sequence encoding a third detectable marker;
(iii) a fourth nucleic acid construct comprising a retroviral transfer vector and a sequence encoding a fourth detectable marker;
which co-expresses the first, second, third (if present) and fourth detectable markers at the cell surface.

The nucleic acid construct(s) may be stably integrated into the cell genome.

The packaging or producer cell may be a HEK293, HEK293-T, TE671 or HT1080, 3T3, or K562 cell.

### METHOD

In one aspect the present invention provides a method for making a packaging cell or producer cell of the invention which comprises the step of introducing one or more nucleic acid construct(s) of the invention, one or more plasmid(s) of the invention, a kit of nucleic acid constructs of the invention, into a cell.

The nucleic acid construct may be introduced into a cell by a variety of methods which are known in the art, for example using standard transfection methods such as electroporation or lipofection.

The nucleic acid constructs may be introduced into the cell during the same step of the method (e.g. transfected at the same time) or at different steps in the method (e.g. transfected at different times).

In another aspect the present invention provides a method for selecting a packaging or producer cell of the invention by selecting for expression of the or each detectable marker encoded by the or each nucleic acid construct wherein cells with optimal expression ratios of different retroviral components are selected from a population of cells.

The method may comprise the following steps:
(a) introducing
   (i) a first nucleic acid construct comprising a *gag-pol* sequence and a sequence encoding a first detectable marker; and
   (ii) a second nucleic acid construct comprising an *env* sequence and a sequence encoding a second detectable marker;
   into a plurality of cells; and
(b) selecting a cell which co-expresses the first and second detectable markers.

Step (a) may further comprise introducing into the plurality of cells:
(iii) a third nucleic acid construct comprising a *rev* sequence and a sequence encoding a third detectable marker;
and step (b) further comprises selecting a cell which co-expresses the first, second and third detectable markers.

Step (a) may further comprise introducing into the plurality of cells:
(iv) a fourth nucleic acid construct comprising a retroviral transfer vector and a sequence encoding a fourth detectable marker;
and step (b) further comprises selecting a cell which co-expresses the first, second, fourth and optionally third detectable markers.

Step (a) may also comprise introducing a nucleic acid sequence encoding piggyBAC transposase.

In another aspect, the present invention provides a method for selecting a packaging cell or producer cell according to the invention by selecting for expression of the or each detectable marker encoded by the or each nucleic acid construct wherein cells with optimal expression ratios of different retroviral components are selected from a population of cells.

In one embodiment, the screening is performed by fluorescence-activated cell sorting (FACS).

For example, by sorting vector, gagpol, env and rev expressing cells using flow cytometry, it is possible to monitor the expression levels of the different retroviral components and select cells with optimal ratios of expression of each component for more productive retroviral production.

The cell may express a plurality of detectable markers and may be selected using multiparameter flow cytometry.

The method may comprise the following steps:
(a) introducing
   (i) a first nucleic acid construct comprising a *gag-pol* sequence and a sequence encoding a first detectable marker;
   (ii) a second nucleic acid construct comprising an *env* sequence and a sequence encoding a second detectable marker;
   into a plurality of cells; and
(b) selecting a cell which co-expresses the first and second detectable markers.

Step (a) may further comprise introducing into the plurality of cells:
(iii) a third nucleic acid construct comprising a *rev* sequence and a sequence encoding a third detectable marker;
and step (b) further comprises selecting a cell which co-expresses the first, second and third detectable markers.

Step (a) may further comprise introducing into the plurality of cells:
(iv) a fourth nucleic acid construct comprising a retroviral transfer vector and a sequence encoding a fourth detectable marker;
and step (b) further comprises selecting a cell which co-expresses the first, second, fourth and optionally third detectable markers.

Step (a) may also comprise introducing a nucleic acid sequence encoding piggyBAC transposase.

In another aspect the invention provides a method for producing a retroviral vector which comprises the steps of culturing a producer cell of the present invention and isolating the retroviral vector.

The producer cell may be cultured using standard culture conditions suitable for the particular cell type used to make the producer cell. During culture, retrovirus vector particles are released into the culture supernatant. The retrovirus vector may be isolated using standard techniques, for example, ultracentrifugation, ultrafiltration or affinity purification.

The retroviral vector may be a lentiviral vector.

Definitions of terms appear throughout the specification. Before the exemplary embodiments are described in more detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "an enzyme" includes a plurality of such candidate agents and equivalents thereof known to those skilled in the art, and so forth.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the term "consisting of'.

The invention will now be further described by way of Examples, which are meant to serve to assist one of ordinary skill in the art in carrying out the invention and are not intended in any way to limit the scope of the invention.

### EXAMPLES

### Example 1 - Construction of cell surface marker proteins

Possible compact surface marker genes are detailed in Figure 2. Coding sequences for well-characterized epitope tags (V5, HA and MYC-tags) were cloned 3' to the coding sequence for a signal peptide. These in turn were cloned to the stalk, TM and a portion of the endodomain of CD8alpha. Alternatively, they were cloned in frame with the GPI-anchor signal of CD52. This resulted in a set of highly compact surface marker proteins which display the epitope on the surface of the cell. This allows easy detection by staining with a fluorescently conjugated antibody. The compact size of these markers keep the transcriptional burden of expressing a marker gene to a minimum. Surface expression means the cell does not have to be lysed to allow detection.

### Example 2 - Expression of cell surface marker proteins in 293T cells

293T cells were co-transfected with a piggyBAC transposase expression plasmid and a lentiviral rev expression plasmid which also co-expressed V5-8 marker gene. Some 293T cells were also transfected with just the rev expression plasmid alone as a control. These latter cells allow determination of the contribution to expression from transient transfection as opposed to permanent insertion. At 10 days, both populations of cells as well as non-transfected controls were stained with a fluorescent antibody which recognizes the V5 epitope tag. These cells were then analysed by flow-cytometery. A clear population of V5-8 expression cells were seen in the transposase co-transfected cells, while expression in the rev expression alone 293T cells was lower (Figure 3). These cell cultures were maintained for 95 days and periodically analysed for v5 epitope expression. This demonstrates the stability of piggyBAC mediated transposition, and the convenience of marker gene in demonstrating stability of a lentiviral element.

### Example 3 - Co-expression of different lentiviral proteins with cell surface marker proteins

Different lentiviral elements require different means to optimally co-express a marker gene. Lentiviral gagpol was tagged in two different ways: HA-8 tag marker gene was co-expressed with a FMD-2A like peptide, or with an IRES sequence (Figure 4). Three lentiviral gagpol expression cassettes were tested: a control without a tag, the 2A-tagged version and the IRES-tagged version. 293T cells transfected with these plasmids were stained with an anti-HA antibody and analysed by flow-cytometry. The tag could be readily detected in both tag-2A and IRES.tag constructs. Lentiviral supernatant was generated by transiently transfecting helper plasmids and transfer vector where the gagpol was supplied by either one of the above three plasmids. Use of the Tag-2A construct resulted in a lower virus titer. This demonstrated that the IRES was an optimal way of tagging gagpol in this experiment.

### Example 4 - Optimal lentiviral helper expression cassette design

The helper cassettes must supply rev, RD-PRO and gagpol. Suggested optimal cassettes are shown in Figure 5. To assist insertion into the packaging cell genome for stable expression, each cassette is flanked by piggyBAC terminal repeats (pB-TR). Promoters, polyadenylation sequences and introns are selected to be as different as possible to reduce the probability of homologous recombination. In this case, rev cassette contiains an RSV promoter, the RD-PRO cassette contains an SV40/Ferritin heavy-chain composite promoter and the gagpol cassette contains a CMV/β-actin composite promoter. Each lentiviral element is co-expressed with a surface epitope marker. Co-expression in the Rev and RD-PRO cassette is achieved with a FMD like 2A peptide. Co-expression in the gagpol cassette is achieved with an IRES. RD-PRO requires an intron for efficient expression. This is provided by the mouse EFalpha 5' UTR. The gagpol cassette contains an interon from rabbit β-globin as well a scaffold attachment region (SAR) to enhance expression.

### Example 5 - Selection of optimal lentiviral producer cells

Figure 1 shows a scheme for generation of a stable lentiviral producer line. 293T cells are co-transfected with lentiviral helper plasmids as described above. A lentiviral transfer vector is also co-transfected. One of the transfer vector transgenes is RQR8 which, like the tags, is easily detected on the surface of the packaging cell. Like the helper plasmids, the transfer vector is flanked by piggyBAC terminal repeats. An expression plasmid supplying the piggyBAC transposase is also co-transfected. After a period of culture, permanent expression of helper and transgene cassettes can easily be detected by flow cytometry. Figure 6 shows 293T stably transposed with lentiviral helper cassettes and a transfer vector. All 4 tags can be independently detected and populations of cells which express desired amounts of the different elements can be gated (an example gate is shown in Figure 6) and flow-sorted. Large numbers of these "bulk" transposed cells are sorted by flow cytometric sorting, with precise population(s) of cells with high transgene and optimal relative expression being selected. These are directly sorted as single-cells by the flow-sorter. They are allowed to expand to fill the well of a 96 or 385 well plate and supernatant is titered. The highest titer clones are selected for virus production.

### Example 6 - Lentiviral vector production from a stably transposed tagged gagpol expression cassette

An expression cassette was generated where lentiviral gagpol could be transposed (the cassette was flanked by piggyBAC terminal repeats - see Figure 10). Further, gagpol was co-expressed with a surface expressed marker gene. 293T cells were co-transfected with this plasmid and an expression plasmid which provided piggyBAC transposase. The 293T cells were cultured for 10 days to allow expression to stabilize (i.e. transient expression from transfection to be lost, leaving only expression from stably transposed cells). By staining for the marker gene, 293T cells were sorted by flow cytometry sorting into low, medium and high expressing cells. These populations of 293T cells were expanded after the sort and were then transfected with RD114 envelop, REV and a transfer vector. Supernatant was harvested and tittered on 293T cells.

Stable transposed gagpol could result in vector production. Low expressing cells generated higher titer. This suggests that careful gating of cell populations possible during flow sorting may help to identify particularly productive cell populations or clones (Figure 7).

### Example 7 - Lentiviral vector production from a stably transposed tagged transfer vector cassette

A lentiviral transfer vector was generated which was tagged with a surface-expressed epitope marker which was flanked by piggyBAC terminal repeats (see Figure 9). 293T cells were co-transfected with this cassette and a second expression caassette which supplied piggyBAC transposase. The 293T cells were cultured for 10 days to allow expression to stabilize (i.e. transient expression to dissipate). By staining for the marker gene, 293T cells could be sorted for cells which were high or low expressing. Cells which were not expressing the marker could be excluded. These 293T cells were cultivated and transfected with plasmids supplying gagpol, RD114 envelop and REV. Supernatant was tittered on 293T cells.

Titration showed that viral vector was produced. Productivity did not correlate with high expression of marker gene (see Figure 8).

## Claims

1. A nucleic acid construct comprising:
(i) a first nucleic acid sequence which encodes a retroviral protein; and
(ii) a second nucleic acid sequence which encodes a detectable marker which is a cell surface protein comprising an extracellular domain and a membrane targeting domain; wherein the nucleic acid construct has the structure:
A-X-B
in which
A is the first nucleic acid sequence, B is the second nucleic acid sequence and X is a co-expression sequence; wherein the co-expression sequence enables the retroviral protein and the detectable marker to be expressed as separate polypeptides.

2. A nucleic acid construct according to claim 1, wherein the co-expression sequence comprises an IRES sequence or a sequence encoding a self-cleaving peptide.

3. A nucleic acid construct according to any preceding claim wherein (a) the first nucleic acid sequence encodes a retroviral protein selected from gag-pol, env or rev; and/or (b) the cell surface protein has less than 200 amino acids; and/or (c) the membrane targeting domain of the cell surface protein comprises (i) a transmembrane domain; or (ii) a GPI anchor; and/or (d) the membrane targeting domain comprises a CD8 stalk or a part thereof; and/or (e) the extracellular domain comprises HA, V5, RQR8 or MYC; and/or (f) the cell surface protein comprises an amino acid sequence as shown as SEQ ID NO: 21-26; and/or (g) comprises transposon sequences flanking the first and second nucleic acid sequence sequences; optionally wherein the nucleic acid construct has the structure:
T1-A-X-B-T2
In which A, X and B are as defined in claim 1;
T1 is a first transposon sequence; and
T2 is a second transposon sequence; further optionally
wherein T1 is a PiggyBAC 5' Terminal Repeat comprising the sequence shown as SEQ ID NO: 60 and T2 is a PiggyBAC 3' Terminal repeat comprising the sequence shown as SEQ ID NO: 61.

4. A kit comprising a plurality of nucleic acid constructs according to any preceding claim.

5. A kit according to claim 4, which comprises:
(i) a first nucleic acid construct comprising a *gag-pol* sequence and a sequence encoding a first detectable marker;
(ii) a second nucleic acid construct comprising an *env* sequence and a sequence encoding a second detectable marker;
in which the first and second detectable markers are cell surface proteins comprising an extracellular domain and a membrane targeting domain and are different to each other; optionally wherein the kit further comprises:
(iii) a third nucleic acid construct comprising a *rev* sequence and a sequence encoding a third detectable marker which is a cell surface protein comprising an extracellular domain and a membrane targeting domain;
in which the first, second and third and detectable markers are all different to each other; and/or wherein the kit further comprises:
(iv) a fourth nucleic acid construct comprising a retroviral transfer vector and a sequence encoding a fourth detectable marker which is a cell surface protein comprising an extracellular domain and a membrane targeting domain;
in which the first, second, third (if present) and fourth detectable markers are all different to each other.

6. A plasmid comprising a nucleic acid construct as defined in any of claims 1 to 3.

7. A packaging cell comprising a nucleic acid construct according to any of claims 1 to 3 which expresses at least one detectable marker which is a cell surface protein comprising an extracellular domain and a membrane targeting domain.

8. A packaging cell according to claim 7 comprising:
(i) a first nucleic acid construct comprising a *gag-pol* sequence and a sequence encoding a first detectable marker;
(ii) a second nucleic acid construct comprising an *env* sequence and a sequence encoding a second detectable marker;
which co-expresses the first and second detectable markers at the cell surface; optionally wherein the packaging cell further comprises:
(iii) a third nucleic acid construct comprising a *rev* sequence and a sequence encoding a third detectable marker;
which co-expresses the first, second and third detectable markers at the cell surface.

9. A producer cell which comprises first and second nucleic acid constructs as defined in claim 8 and optionally a third nucleic acid construct as defined in claim 8 and which further comprises:
a fourth nucleic acid construct comprising a retroviral transfer vector and a sequence encoding a fourth detectable marker;
which co-expresses the first, second, third (if present) and fourth detectable markers at the cell surface.

10. A packaging cell according to claims 7 or 8 or a producer cell according to claim 9 wherein the or each nucleic acid construct is/are stably integrated into the cell genome.

11. A method for making a packaging cell or producer cell according to any of claims 7 to 10 which comprises the step of introducing one or more nucleic acid construct(s) as defined in any of claims 1 to 3, one or more plasmid(s) as defined in claim 6, or a plurality of nucleic acid constructs as defined in the kit according to claims 4 or 5, into a cell.

12. A method for selecting a packaging cell or producer cell according to any of claims 7 to 10 by selecting for expression of the or each detectable marker encoded by the or each nucleic acid construct; wherein cells with optimal expression ratios of different retroviral components are selected from a population of cells; optionally
wherein the cell expresses a plurality of detectable markers and the cell is selected using multi-parameter flow cytometry; and/or wherein the method comprises the following steps:
(a) introducing
(i) a first nucleic acid construct comprising a *gag-pol* sequence and a sequence encoding a first detectable marker;
(ii) a second nucleic acid construct comprising an *env* sequence and a sequence encoding a second detectable marker;
into a plurality of cells; and
(b) selecting a cell which co-expresses the first and second detectable markers; optionally wherein step (a) further comprises introducing into the plurality of cells:
(iii) a third nucleic acid construct comprising a *rev* sequence and a sequence encoding a third detectable marker;
and step (b) further comprises selecting a cell which co-expresses the first, second and third detectable markers; and/or wherein step (a) further comprises introducing into the plurality of cells: (iv) a fourth nucleic acid construct comprising a retroviral transfer vector and a sequence encoding a fourth detectable marker;
and step (b) further comprises selecting a cell which co-expresses the first, second, fourth and optionally third detectable markers; and/or wherein step (a) further comprises introducing a nucleic acid sequence encoding piggyBAC transposase.

13. A method for producing a retroviral vector which comprises the steps of culturing a producer cell according to claim 9 and isolating the retroviral vector.

14. A method for producing a retroviral vector which comprises the steps of introducing a retroviral vector genome into a packaging cell according to any of claims 7, 8, or 10 and isolating the retroviral vector.

15. A method according to claim 13 or 14 wherein the retroviral vector is a lentiviral vector.

## Patentansprüche

1. Nukleinsäurekonstrukt, umfassend:
(i) eine erste Nukleinsäuresequenz, die ein retrovirales Protein codiert; und
(ii) eine zweite Nukleinsäuresequenz, die einen nachweisbaren Marker codiert, bei dem es sich um ein Zelloberflächenprotein handelt, das eine extrazelluläre Domäne und eine Membran-Targeting-Domäne umfasst;
wobei das Nukleinsäurekonstrukt die Struktur
A-X-B
aufweist, bei der
A für die erste Nukleinsäuresequenz steht, B für die zweite Nukleinsäuresequenz steht und X für eine Coexpressionssequenz steht; wobei die Coexpressionssequenz die Expression des retroviralen Proteins und des nachweisbaren Markers als getrennte Polypeptide ermöglicht.

2. Nukleinsäurekonstrukt nach Anspruch 1, wobei die Coexpressionssequenz eine IRES-Sequenz oder eine Sequenz, die ein selbstspaltendes Peptid codiert, umfasst.

3. Nukleinsäurekonstrukt nach einem vorhergehenden Anspruch, wobei (a) die erste Nukleinsäuresequenz ein retrovirales Protein ausgewählt aus gag-pol, env oder rev codiert; und/oder (b) das Zelloberflächenprotein weniger als 200 Aminosäuren aufweist; und/oder (c) die Membran-Targeting-Domäne des Zelloberflächenproteins (i) eine Transmembrandomäne; oder (ii) einen GPI-Anker umfasst; und/oder (d) die Membran-Targeting-Domäne einen CD8-Stiel oder einen Teil davon umfasst; und/oder (e) die extrazelluläre Domäne HA, V5, RQR8 oder MYC umfasst; und/oder (f) das Zelloberflächenprotein eine Aminosäuresequenz gemäß SEQ ID NO: 21-26 umfasst; und/oder (g) Transposonsequenzen umfasst, die die Sequenzen der ersten und zweiten Nukleinsäuresequenz flankieren; gegebenenfalls wobei das Nukleinsäurekonstrukt die Struktur
T1-A-X-B-T2
aufweist, bei der A, X und B die in Anspruch 1 angegebene Bedeutung haben;
T1 für eine erste Transposonsequenz steht; und
T2 für eine zweite Transposonsequenz steht; ferner gegebenenfalls
wobei T1 für eine PiggyBAC 5' Terminal Repeat steht, die die Sequenz gemäß SEQ ID NO: 60 umfasst, und T2 für eine PiggyBAC 3' Terminal Repeat steht, die die Sequenz gemäß SEQ ID NO: 61 umfasst.

4. Kit, umfassend mehrere Nukleinsäurekonstrukte nach einem vorhergehenden Anspruch.

5. Kit nach Anspruch 4, das Folgendes umfasst:
(i) ein erstes Nukleinsäurekonstrukt, das eine *gag-pol-*Sequenz und eine Sequenz, die einen ersten nachweisbaren Marker codiert, umfasst;
(ii) ein zweites Nukleinsäurekonstrukt, das eine *env-*Sequenz und eine Sequenz, die einen zweiten nachweisbaren Marker codiert, umfasst;
bei dem es sich bei dem ersten und dem zweiten nachweisbaren Marker um Zelloberflächenproteine handelt, die eine extrazelluläre Domäne und eine Membran-Targeting-Domäne umfassen und voneinander verschieden sind;
gegebenenfalls wobei das Kit ferner Folgendes umfasst:
(iii) ein drittes Nukleinsäurekonstrukt, das eine rev-Sequenz und eine Sequenz, die einen dritten nachweisbaren Marker codiert, bei dem es sich um ein Zelloberflächenprotein handelt, das eine extrazelluläre Domäne und eine Membran-Targeting-Domäne umfasst, umfasst;
bei dem der erste, zweite und dritte nachweisbare Marker jeweils voneinander verschieden sind; und/oder wobei das Kit ferner Folgendes umfasst:
(iv) ein viertes Nukleinsäurekonstrukt, das einen retroviralen Transfervektor und eine Sequenz, die einen vierten nachweisbaren Marker codiert, bei dem es sich um ein Zelloberflächenprotein handelt, das eine extrazelluläre Domäne und eine Membran-Targeting-Domäne umfasst, umfasst;
bei dem der erste, zweite, dritte (falls vorhanden) und vierte nachweisbare Marker jeweils voneinander verschieden sind.

6. Plasmid, umfassend ein Nukleinsäurekonstrukt gemäß einem der Ansprüche 1 bis 3.

7. Verpackungszelle, umfassend ein Nukleinsäurekonstrukt nach einem der Ansprüche 1 bis 3, die wenigstens einen nachweisbaren Marker exprimiert, bei dem es sich um ein Zelloberflächenprotein handelt, das eine extrazelluläre Domäne und eine Membran-Targeting-Domäne umfasst.

8. Verpackungszelle nach Anspruch 7, umfassend:
(i) ein erstes Nukleinsäurekonstrukt, das eine *gag-pol-*Sequenz und eine Sequenz, die einen ersten nachweisbaren Marker codiert, umfasst;
(ii) ein zweites Nukleinsäurekonstrukt, das eine *env-*Sequenz und eine Sequenz, die einen zweiten nachweisbaren Marker codiert, umfasst;
die den ersten und den zweiten nachweisbaren Marker an der Zelloberfläche coexprimiert; gegebenenfalls wobei die Verpackungszelle ferner Folgendes umfasst:
(iii) ein drittes Nukleinsäurekonstrukt, das eine rev-Sequenz und eine Sequenz, die einen dritten nachweisbaren Marker codiert, umfasst;
die den ersten, zweiten und dritten nachweisbaren Marker an der Zelloberfläche coexprimiert.

9. Produktionszelle, die erste und zweite Nukleinsäurekonstrukte gemäß Anspruch 8 und gegebenenfalls ein drittes Nukleinsäurekonstrukt gemäß Anspruch 8 umfasst und die ferner Folgendes umfasst:
ein viertes Nukleinsäurekonstrukt, das einen retroviralen Transfervektor und eine Sequenz, die einen vierten nachweisbaren Marker codiert, umfasst;
die den ersten, zweiten, dritten (falls vorhanden) und vierten nachweisbaren Marker an der Zelloberfläche coexprimiert.

10. Verpackungszelle nach Anspruch 7 oder 8 oder Produktionszelle nach Anspruch 9, wobei das bzw. jedes Nukleinsäurekonstrukt in das Zellgenom stabil integriert wird.

11. Verfahren zur Erzeugung einer Verpackungszelle oder Produktionszelle nach einem der Ansprüche 7 bis 10, das den Schritt umfasst, bei dem ein oder mehrere Nukleinsäurekonstrukt(e) gemäß einem der Ansprüche 1 bis 3, ein oder mehrere Plasmid(e) gemäß Anspruch 6 oder mehrere Nukleinsäurekonstrukte gemäß dem Kit nach Anspruch 4 oder 5 in eine Zelle eingeführt werden.

12. Verfahren zum Selektieren einer Verpackungszelle oder Produktionszelle nach einem der Ansprüche 7 bis 10 durch Selektieren auf Expression des oder jedes nachweisbaren Markers, der durch das bzw. das jeweilige Nukleinsäurekonstrukt codiert ist; wobei Zellen mit optimalen Expressionsverhältnissen unterschiedlicher retroviraler Komponenten aus einer Population von Zellen selektiert werden; gegebenenfalls
wobei die Zelle mehrere nachweisbare Marker exprimiert und die Zelle unter Verwendung von Multiparameter-Durchflusszytometrie selektiert wird; und/oder wobei das Verfahren die folgenden Schritte umfasst:
(a) Einführen
(i) eines ersten Nukleinsäurekonstrukts, das eine gag-pol-Sequenz und eine Sequenz, die einen ersten nachweisbaren Marker codiert, umfasst;
(ii) eines zweiten Nukleinsäurekonstrukts, das eine *env-*Sequenz und eine Sequenz, die einen zweiten nachweisbaren Marker codiert, umfasst;
in mehrere Zellen; und
(b) Selektieren einer Zelle, die den ersten und den zweiten nachweisbaren Marker coexprimiert;
gegebenenfalls wobei Schritt (a) ferner Einführen
(iii) eines dritten Nukleinsäurekonstrukts, das eine rev-Sequenz und eine Sequenz, die einen dritten nachweisbaren Marker codiert, umfasst, in die mehreren Zellen umfasst; und Schritt (b) ferner Selektieren einer Zelle, die den ersten, zweiten und dritten nachweisbaren Marker coexprimiert, umfasst; und/oder wobei Schritt (a) ferner Einführen (iv) eines vierten Nukleinsäurekonstrukts, das einen retroviralen Transfervektor und eine Sequenz, die einen vierten nachweisbaren Marker codiert, umfasst, in die mehreren Zellen umfasst;
und Schritt (b) ferner Selektieren einer Zelle, die den ersten, zweiten, vierten und gegebenenfalls dritten nachweisbaren Marker coexprimiert, umfasst; und/oder wobei Schritt (a) ferner Einführen einer Nukleinsäuresequenz, die piggyBAC-Transposase codiert, umfasst.

13. Verfahren zur Herstellung eines Retrovirusvektors, das die Schritte Kultivieren einer Produktionszelle nach Anspruch 9 und Isolieren des retroviralen Vektors umfasst.

14. Verfahren zur Produktion eines Retrovirusvektors, das die Schritte Einführen eines Retrovirusvektorgenoms in eine Verpackungszelle nach einem der Ansprüche 7, 8 oder 10 und Isolieren des Retrovirusvektors umfasst.

15. Verfahren nach Anspruch 13 oder 14, wobei es sich bei dem Retrovirusvektor um Lentivirusvektor handelt.

## Revendications

1. Construction d'acides nucléiques comprenant :
(i) une première séquence d'acides nucléiques qui code pour une protéine rétrovirale ; et
(ii) une deuxième séquence d'acides nucléiques qui code pour un marqueur détectable, qui est une protéine de surface cellulaire comprenant un domaine extracellulaire et un domaine de ciblage membranaire ; la construction d'acides nucléiques ayant la structure :
A-X-B
dans laquelle
A est la première séquence d'acides nucléiques, B est la deuxième séquence d'acides nucléiques et X est une séquence d'expression conjointe ; dans laquelle la séquence d'expression conjointe permet que la protéine rétrovirale et le marqueur détectable soient exprimés en tant que polypeptides séparés.

2. Construction d'acides nucléiques selon la revendication 1, dans laquelle la séquence d'expression conjointe comprend une séquence IRES ou une séquence codant pour un peptide s'auto-coupant.

3. Construction d'acides nucléiques selon une quelconque revendication précédente, dans laquelle (a) la première séquence d'acides nucléiques qui code pour une protéine rétrovirale choisie parmi gag-pol, env ou rev ; et/ou (b) la protéine de surface cellulaire a moins de 200 acides aminés ; et/ou (c) le domaine de ciblage membranaire de la protéine de surface cellulaire comprend (i) un domaine transmembranaire ; ou (ii) une ancre de GPI ; et/ou (d) le domaine de ciblage membranaire comprend une tige de CD8 ou une partie de celle-ci ; et/ou (e) le domaine extracellulaire comprend HA, V5, RQR8 ou MYC ; et/ou (f) la protéine de surface cellulaire comprend une séquence d'acides aminés telle que présentée comme SEQ ID n° : 21-26 ; et/ou (g) comprend des séquences transposons flanquant les première et deuxième séquences d'acides nucléiques ; en option, la construction d'acides nucléiques ayant la structure :
T1-A-X-B-T2
dans laquelle A, X et B sont tels que définis dans la revendication 1 ;
T1 est une première séquence transposon ; et
T2 est une deuxième séquence transposon ; en outre en option
dans laquelle T1 est une répétition terminale en 5' PiggyBAC, comprenant la séquence présentée comme SEQ ID n° : 60, et T2 est une répétition terminale en 3' PiggyBAC, comprenant la séquence présentée comme SEQ ID n° : 61.

4. Trousse comprenant une pluralité de constructions d'acides nucléiques selon une quelconque revendication précédente.

5. Trousse selon la revendication 4, qui comprend :
(i) une première construction d'acides nucléiques comprenant une séquence *gag-pol* et une séquence codant pour un premier marqueur détectable ;
(ii) une deuxième construction d'acides nucléiques comprenant une séquence *env* et une séquence codant pour un deuxième marqueur détectable ;
dans laquelle les premier et deuxième marqueurs détectables sont des protéines de surface cellulaire comprenant un domaine extracellulaire et un domaine de ciblage membranaire, et sont différents l'un de l'autre ;
en option la trousse comprenant en outre :
(iii) une troisième construction d'acides nucléiques comprenant une séquence *rev* et une séquence codant pour un troisième marqueur détectable, qui est une protéine de surface cellulaire comprenant un domaine extracellulaire et un domaine de ciblage membranaire ; dans laquelle les premier, deuxième et troisième marqueurs détectables sont tous différents les uns des autres ;
et/ou la trousse comprenant en outre :
(iv) une quatrième construction d'acides nucléiques comprenant un vecteur de transfert rétroviral et une séquence codant pour un quatrième marqueur détectable, qui est une protéine de surface cellulaire comprenant un domaine extracellulaire et un domaine de ciblage membranaire ;
dans laquelle les premier, deuxième, troisième (s'il est présent) et quatrième marqueurs détectables sont tous différents les uns des autres.

6. Plasmide comprenant une construction d'acides nucléiques telle que définie dans l'une quelconque des revendications 1 à 3.

7. Cellule d'empaquetage comprenant une construction d'acides nucléiques, selon l'une quelconque des revendications 1 à 3, qui exprime au moins un marqueur détectable qui est une protéine de surface cellulaire comprenant un domaine extracellulaire et un domaine de ciblage membranaire.

8. Cellule d'empaquetage selon la revendication 7, comprenant :
(i) une première construction d'acides nucléiques comprenant une séquence *gag-pol* et une séquence codant pour un premier marqueur détectable ;
(ii) une deuxième construction d'acides nucléiques comprenant une séquence *env* et une séquence codant pour un deuxième marqueur détectable ;
qui exprime conjointement les premier et deuxième marqueurs détectables à la surface de la cellule ; en option, la cellule d'empaquetage comprenant en outre :
(iii) une troisième construction d'acides nucléiques comprenant une séquence *rev* et une séquence codant pour un troisième marqueur détectable ;
qui exprime conjointement les premier, deuxième et troisième marqueurs détectables à la surface de la cellule.

9. Cellule productrice qui comprend des première et deuxième constructions d'acides nucléiques, telles que définies dans la revendication 8, et en option une troisième construction d'acides nucléiques, telle que définie dans la revendication 8, et qui comprend en outre :
une quatrième construction d'acides nucléiques comprenant un vecteur de transfert rétroviral et une séquence codant pour un quatrième marqueur détectable ;
qui exprime conjointement les premier, deuxième, troisième (s'il est présent) et quatrième marqueurs détectables à la surface de la cellule.

10. Cellule d'empaquetage, selon les revendications 7 ou 8, ou cellule productrice, selon la revendication 9, dans laquelle la ou chaque construction d'acides nucléiques est stablement intégrée dans le génome de la cellule.

11. Procédé destiné à élaborer une cellule d'empaquetage ou une cellule productrice, selon l'une quelconque des revendications 7 à 10, qui comprend l'étape d'introduction d'une ou de plusieurs construction(s) d'acides nucléiques telles que définies dans l'une quelconque des revendications 1 à 3, d'un ou de plusieurs plasmide(s) tels que définis dans la revendication 6 ou d'une pluralité de constructions d'acides nucléiques telles que définies dans la trousse selon les revendications 4 ou 5, dans une cellule.

12. Procédé destiné à sélectionner une cellule d'empaquetage ou une cellule productrice, selon l'une quelconque des revendications 7 à 10, en faisant une sélection en vue de l'expression du ou de chaque marqueur détectable codé par la ou chaque construction d'acides nucléiques ; dans lequel les cellules avec des rapports d'expression optimaux de différents composants rétroviraux sont sélectionnées à partir d'une population de cellules ; en option
dans lequel la cellule exprime une pluralité de marqueurs détectables et la cellule est sélectionnée en utilisant une cytométrie en flux multiparamétrique ; et/ou le procédé comprenant les étapes suivantes :
(a) introduction
(i) d'une première construction d'acides nucléiques comprenant une séquence *gag-pol* et une séquence codant pour un premier marqueur détectable ;
(ii) d'une deuxième construction d'acides nucléiques comprenant une séquence *env* et une séquence codant pour un deuxième marqueur détectable ;
dans une pluralité de cellules ; et
(b) sélection d'une cellule qui exprime conjointement les premier et deuxième marqueurs détectables ; en option, dans lequel l'étape (a) comprend en outre une introduction dans la pluralité de cellules :
(iii) d'une troisième construction d'acides nucléiques comprenant une séquence *rev* et une séquence codant pour un troisième marqueur détectable ;
et l'étape (b) comprend en outre une sélection d'une cellule qui exprime conjointement les premier, deuxième et troisième marqueurs détectables ; et/ou dans lequel l'étape (a) comprend en outre une introduction dans la pluralité de cellules : (iv) d'une quatrième construction d'acides nucléiques comprenant un vecteur de transfert rétroviral et une séquence codant pour un quatrième marqueur détectable ;
et l'étape (b) comprend en outre une sélection d'une cellule qui exprime conjointement le premier, le deuxième, le quatrième et, en option, le troisième marqueurs détectables ; et/ou dans lequel l'étape (a) comprend en outre une introduction d'une séquence d'acides nucléiques codant pour une transposase de piggyBAC.

13. Procédé destiné à produire un vecteur rétroviral qui comprend les étapes de culture d'une cellule productrice, selon la revendication 9, et d'isolement du vecteur rétroviral.

14. Procédé destiné à produire un vecteur rétroviral qui comprend les étapes d'introduction d'un génome de vecteur rétroviral dans une cellule d'empaquetage, selon l'une quelconque des revendications 7, 8 ou 10, et d'isolement du vecteur rétroviral.

15. Procédé selon les revendications 13 ou 14, dans lequel le vecteur rétroviral est un vecteur lentiviral.


### SEQUENCE LISTING

<110> UCL Business PLC
<120> CELL
<130> P108338PCT
<150> GB 1603372.2
   <151> 2016-02-26
<160> 61
<170> PatentIn version 3.5
<210> 1
   <211> 20
   <212> PRT
   <213> Artificial Sequence
<220>
   <223> signal peptide
<400> 1
   Met Glu Thr Asp Thr Leu Leu Leu Trp Val Leu Leu Leu Trp Val Pro
   1               5                   10                  15
   Gly Ser Thr Gly
               20
<210> 2
   <211> 21
   <212> PRT
   <213> Artificial Sequence
<220>
   <223> signal peptide
<400> 2
   Met Gly Thr Ser Leu Leu Cys Trp Met Ala Leu Cys Leu Leu Gly Ala
   1               5                   10                  15
   Asp His Ala Asp Gly
               20
<210> 3
   <211> 4
   <212> PRT
   <213> Artificial Sequence
<220>
   <223> basic amino acid furin target sequence
<220>
   <221> misc_feature
   <222> (2)..(2)
   <223> Xaa can be any naturally occurring amino acid
<220>
   <221> MISC_FEATURE
   <222> (3)..(3)
   <223> Xaa may be Arg or Lys
<400> 3
   Arg Xaa Xaa Arg
   1
<210> 4
   <211> 7
   <212> PRT
   <213> Artificial Sequence
<220>
   <223> consensus Tobacco Etch Virus (TEV) cleavage site
<400> 4
   Glu Asn Leu Tyr Phe Gln Ser
   1 5
<210> 5
   <211> 8
   <212> PRT
   <213> Artificial Sequence
<220>
   <223> cleavage site
<220>
   <221> misc_feature
   <222> (2)..(2)
   <223> Xaa can be any naturally occurring amino acid
<220>
   <221> misc_feature
   <222> (4)..(4)
   <223> Xaa can be any naturally occurring amino acid
<400> 5
   Asp Xaa Glu Xaa Asn Pro Gly Pro
   1 5
<210> 6
   <211> 8
   <212> PRT
   <213> Artificial Sequence
<220>
   <223> cleavage site
<400> 6
   Asp Ile Glu Thr Asn Pro Gly Pro
   1 5
<210> 7
   <211> 8
   <212> PRT
   <213> Artificial Sequence
<220>
   <223> cleavage site
<400> 7
   Asp Val Glu Thr Asn Pro Gly Pro
   1 5
<210> 8
   <211> 8
   <212> PRT
   <213> Artificial Sequence
<220>
   <223> cleavage site
<220>
   <221> MISC_FEATURE
   <222> (2)..(2)
   <223> Xaa may be Ile, Val, Met or Ser
<220>
   <221> MISC_FEATURE
   <222> (4)..(4)
   <223> Xaa may be Thr, Met, Ser, Leu, Glu, Gln or Phe
<400> 8
   Asp Xaa Glu Xaa Asn Pro Gly Pro
   1 5
<210> 9
   <211> 4307
   <212> DNA
   <213> Artificial Sequence
<220>
   <223> nucleic acid sequence encoding the retroviral protein gagpol
<400> 9
   atgggagcac gggctagtgt tctttctgga ggtgagcttg acaggtggga gaagatcaga      60
   ctgcgccccg gcggcaaaaa gaagtacaag ctgaagcaca tcgtgtgggc ctctcgcgaa     120
   ttggagaggt ttgccgtgaa ccccgggctc ctggagacaa gcgaggggtg ccggcagatc     180
   ctcggccaat tgcagcccag tttgcaaacc ggcagcgagg agttgcggag cctgtacaac     240
   accgtggcca cattgtactg cgtccaccag cgcatcgaaa tcaaggatac aaaagaggcc     300
   ctggataaaa tcgaagagga acagaataag agcaaaaaga aggcccaaca agccgccgct     360
   gataccggcc attctaacca agtgtctcag aactatccca tcgtccaaaa tattcaaggc 420
   cagatggtcc accaagctat cagcccccgg accctgaacg cctgggtgaa ggtggtggag 480
   gaaaaagcct tttctcccga ggtcatccct atgttcagcg ccctgagcga gggcgctaca 540
   ccccaggacc tgaatacaat gttgaatacc gtcggcggcc accaggccgc tatgcagatg 600
   ctgaaggaaa caattaacga agaggccgcc gagtgggacc gggtccaccc cgtccaggct 660
   ggccccatcg cacccgggca aatgcgggag ccgagaggct ccgatatcgc cggcaccacc 720
   tccacattgc aagagcagat cggctggatg accaacaatc ccccaattcc cgtgggcgag 780
   atctacaagc ggtggatcat tctcggcctg aacaagatcg tgcggatgta ctctcccaca 840
   tctatcctcg atatccggca gggccccaaa gagcctttcc gggattacgt ggatagattt 900
   tacaagacct tgcgggctga acaggccagc caagaagtga agaactggat gacggagaca 960
   ctcctcgtgc agaacgccaa tcccgactgc aaaaccatcc tgaaggcctt gggcccagcc 1020
   gccaccttgg aggagatgat gaccgcctgc caaggcgtgg gaggccctgg gcacaaagcc 1080
   cgggtgctcg ccgaggccat gtctcaggtg accaacagcg ccacaatcat gatgcaacgg 1140
   gggaacttcc gcaatcagcg gaaaatcgtg aaatgcttta actgtggcaa agaagggcac 1200
   acagcccgca actgcagggc ccctaggaaa aagggctgtt ggaaatgtgg aaaggaagga 1260
   caccaaatga aagattgtac tgagagacag gctaattttt tagggaagat ctggccttcc 1320
   cacaagggaa ggccagggaa ttttcttcag agcagaccag agccaacagc cccaccagaa 1380
   gagagcttca ggtttgggga agagacaaca actccctctc agaagcagga gccgatagac 1440
   aaggaactgt atcctttagc ttccctcaga tcactctttg gcagcgaccc ctcgtcacaa 1500
   taaagatagg ggggcagctc aaggaggctc tcctggccac cggagcagac gacaccgtgc 1560
   tggaggagat gtcactccct ggtcggtgga agcctaagat gattggtggt atagggggct 1620
   tcattaaggt gcggcaatac gaccaaatct tgatcgagat ttgcggccac aaggccatcg 1680
   gcaccgtgct ggtgggcccc acccccgtga atatcatcgg ccggaacctc ctcacccaaa 1740
   tcggctgtac cctgaacttc cctatctctc ccatcgaaac cgtgcccgtg aagctgaaac 1800
   ccggcatgga cgggcccaag gtgaagcagt ggcccctcac cgaggagaag atcaaggccc 1860
   tggtggagat ctgcaccgaa atggagaaag agggcaagat cagcaagatc ggccccgaga 1920
   acccctataa cacccccgtg ttcgctatca aaaagaagga ttccaccaag tggcggaagc 1980
   tggtggactt tcgggagttg aacaaacgga cccaggattt ttgggaggtg cagctgggca 2040
   tcccccaccc tgccggcctg aagaaaaaga agagcgtgac cgtgctcgac gtcggcgacg 2100
   cctacttcag cgtgcctctg gacgaggatt ttcgcaaata caccgccttc acaatcccct 2160
   ccatcaataa cgaaaccccc ggcatccggt accaatataa cgtcttgccc caaggctgga 2220
   agggcagccc cgccatcttt cagtcctcta tgaccaagat tctggaaccc ttccggaagc 2280
   agaaccccga tatcgtgatt taccagtata tggacgacct ctacgtgggc agcgatctgg 2340
   agatcggcca acaccggacc aagatcgaag aactccggca gcacctcctc cgctggggct 2400
   tgacaacccc cgataagaag caccaaaagg agcctccctt tttgtggatg ggctacgagt 2460
   tgcaccccga caagtggacc gtgcaaccca tcgtcctccc cgagaaggat tcttggaccg 2520
   tgaacgatat ccaaaaactg gtcggcaagc tcaactgggc ctcccaaatc tatcccggca 2580
   tcaaggtgcg ccagctgtgc aagttgttgc ggggcacaaa ggcgttgacc gaggtgatcc 2640
   ccttgaccga ggaggccgaa ttggagctcg ccgagaatcg ggaaatcttg aaggagcccg 2700
   tgcacggcgt ctactacgat cccagcaagg atctgatcgc cgagatccaa aaacaaggcc 2760
   aggggcagtg gacctaccag atctaccagg aacccttcaa gaacctcaag accggcaagt 2820
   acgcccggat gagaggcgcc cataccaacg acgtgaagca gctgaccgaa gccgtccaga 2880
   agatcacaac cgagtctatc gtgatctggg gcaaaacccc caagttcaag ctccctatcc 2940
   agaaggaaac gtgggaaacc tggtggaccg aatactggca ggctacatgg attcccgaat 3000
   gggagttcgt gaacacaccc cctctggtca agctgtggta tcaactggaa aaggagccta 3060
   tcgtgggcgc cgagacattt tacgtggacg gcgctgccaa tcgcgaaacc aagctgggca 3120
   aggccggcta cgtgaccaat cggggccgcc agaaggtggt gacattgacc gataccacca 3180
   accagaaaac cgaactgcag gccatctact tggccctcca agacagcggc ctggaggtga 3240
   atatcgtgac cgatagccag tacgccctgg gcattatcca ggcccagccc gaccagtccg 3300
   agagcgaact ggtgaaccag atcatcgaac aactgatcaa gaaagagaaa gtgtacctcg 3360
   cctgggtgcc cgcccataag gggatcggcg gcaacgagca ggtggacaag ctggtgtccg 3420
   ccggcattcg caaggtgttg ttcctggacg gcatcgacaa agctcaggac gagcacgaaa 3480
   agtaccattc caactggcgg gccatggcct ccgacttcaa tttgccaccc gtggtggcca 3540
   aggagatcgt ggcttcttgc gacaagtgcc aattgaaggg cgaggctatg cacggccagg 3600
   tggattgctc ccccggcatc tggcagttgg actgcaccca cctggagggc aaggtgattc 3660
   tcgtggccgt gcacgtggct tccggctaca tcgaggctga ggtgatcccg gccgaaaccg 3720
   gccaagagac tgcctacttc ttgctgaagc tggccggcag gtggcccgta aagaccatcc 3780
   acaccgataa cgggtctaac tttacatccg ccaccgtgaa agctgcttgc tggtgggcag 3840
   gcattaaaca agagttcggc atcccttata accctcagtc ccagggcgtg gtggagagca 3900
   tgaacaagga gctgaaaaag atcatcggcc aagtgcggga ccaagccgag cacttgaaaa 3960
   ccgccgtgca gatggccgtg tttattcata acttcaagcg gaagggcggc atcggcggct 4020
   attccgccgg tgagcggatc gtggatatca tcgccaccga tatccagacc aaggagctgc 4080
   agaagcagat caccaagatc cagaacttca gagtgtacta tcgcgattct cggaacccct 4140
   tgtggaaggg gccagccaaa ttgttgtgga agggggaggg cgccgtggtg atccaggaca 4200
   actccgatat caaggtggtc ccgcggagga aggccaaaat tatccgcgac tacggcaagc 4260
   aaatggccgg cgacgactgc gtcgcctccc ggcaagacga ggactga 4307
<210> 10
   <211> 511
   <212> PRT
   <213> Artificial Sequence
<220>
   <223> retroviral env protein sequence, VSVG env
<400> 10
   Met Lys Cys Leu Leu Tyr Leu Ala Phe Leu Phe Ile Gly Val Asn Cys
   1               5                   10                  15
   Lys Phe Thr Ile Val Phe Pro His Asn Gln Lys Gly Asn Trp Lys Asn
               20                  25                  30
   Val Pro Ser Asn Tyr His Tyr Cys Pro Ser Ser Ser Asp Leu Asn Trp
           35                  40                  45
   His Asn Asp Leu Ile Gly Thr Ala Leu Gln Val Lys Met Pro Lys Ser
       50                  55                  60
   His Lys Ala Ile Gln Ala Asp Gly Trp Met Cys His Ala Ser Lys Trp
   65                  70                  75                  80
   Val Thr Thr Cys Asp Phe Arg Trp Tyr Gly Pro Lys Tyr Ile Thr His
                   85                  90                  95
   Ser Ile Arg Ser Phe Thr Pro Ser Val Glu Gln Cys Lys Glu Ser Ile
               100                 105                 110
   Glu Gln Thr Lys Gln Gly Thr Trp Leu Asn Pro Gly Phe Pro Pro Gln
           115                 120                 125
   Ser Cys Gly Tyr Ala Thr Val Thr Asp Ala Glu Ala Val Ile Val Gln
       130                 135                 140
   Val Thr Pro His His Val Leu Val Asp Glu Tyr Thr Gly Glu Trp Val
   145                 150                 155                 160
   Asp Ser Gln Phe Ile Asn Gly Lys Cys Ser Asn Tyr Ile Cys Pro Thr
                   165                 170                 175
   Val His Asn Ser Thr Thr Trp His Ser Asp Tyr Lys Val Lys Gly Leu
               180                 185                 190
   Cys Asp Ser Asn Leu Ile Ser Met Asp Ile Thr Phe Phe Ser Glu Asp
           195                 200                 205
   Gly Glu Leu Ser Ser Leu Gly Lys Glu Gly Thr Gly Phe Arg Ser Asn
       210                 215                 220
   Tyr Phe Ala Tyr Glu Thr Gly Gly Lys Ala Cys Lys Met Gln Tyr Cys
   225                 230                 235                 240
   Lys His Trp Gly Val Arg Leu Pro Ser Gly Val Trp Phe Glu Met Ala
                   245                 250                 255
   Asp Lys Asp Leu Phe Ala Ala Ala Arg Phe Pro Glu Cys Pro Glu Gly
               260                 265                 270
   Ser Ser Ile Ser Ala Pro Ser Gln Thr Ser Val Asp Val Ser Leu Ile
           275                 280                 285
   Gln Asp Val Glu Arg Ile Leu Asp Tyr Ser Leu Cys Gln Glu Thr Trp
       290                 295                 300
   Ser Lys Ile Arg Ala Gly Leu Pro Ile Ser Pro Val Asp Leu Ser Tyr
   305                 310                 315                 320
   Leu Ala Pro Lys Asn Pro Gly Thr Gly Pro Ala Phe Thr Ile Ile Asn
                   325                 330                 335
   Gly Thr Leu Lys Tyr Phe Glu Thr Arg Tyr Ile Arg Val Asp Ile Ala
               340                 345                 350
   Ala Pro Ile Leu Ser Arg Met Val Gly Met Ile Ser Gly Thr Thr Thr
           355                 360                 365
   Glu Arg Glu Leu Trp Asp Asp Trp Ala Pro Tyr Glu Asp Val Glu Ile
       370                 375                 380
   Gly Pro Asn Gly Val Leu Arg Thr Ser Ser Gly Tyr Lys Phe Pro Leu
   385                 390                 395                 400
   Tyr Met Ile Gly His Gly Met Leu Asp Ser Asp Leu His Leu Ser Ser
                   405                 410                 415
   Lys Ala Gln Val Phe Glu His Pro His Ile Gln Asp Ala Ala Ser Gln
               420                 425                 430
   Leu Pro Asp Asp Glu Ser Leu Phe Phe Gly Asp Thr Gly Leu Ser Lys
               435                 440                 445
   Asn Pro Ile Glu Leu Val Glu Gly Trp Phe Ser Ser Trp Lys Ser Ser
       450                 455                 460
   Ile Ala Ser Phe Phe Phe Ile Ile Gly Leu Ile Ile Gly Leu Phe Leu
   465                 470                 475                 480
   Val Leu Arg Val Gly Ile His Leu Cys Ile Lys Leu Lys His Thr Lys
                   485                 490                 495
   Lys Arg Gln Ile Tyr Thr Asp Ile Glu Met Asn Arg Leu Gly Lys
               500                 505                 510
<210> 11
<400> 11
   000
<210> 12
   <211> 27
   <212> PRT
   <213> Artificial Sequence
<220>
   <223> CD28 transmembrane domain
<400> 12
   Phe Trp Val Leu Val Val Val Gly Gly Val Leu Ala Cys Tyr Ser Leu
   1               5                   10                  15
   Leu Val Thr Val Ala Phe Ile Ile Phe Trp Val
               20                  25
<210> 13
   <211> 25
   <212> PRT
   <213> Artificial Sequence
<220>
   <223> GPI signal sequence
<400> 13
   Ala Ser Ser Asn Ile Ser Gly Gly Ile Phe Leu Phe Phe Val Ala Asn
   1               5                   10                  15
   Ala Ile Ile His Leu Phe Cys Phe Ser
               20                  25
<210> 14
   <211> 9
   <212> PRT
   <213> Artificial Sequence
<220>
   <223> Human influenza hemagglutinin (HA) tag
<400> 14
   Tyr Pro Tyr Asp Val Pro Asp Tyr Ala
   1               5
<210> 15
   <211> 14
   <212> PRT
   <213> Artificial Sequence
<220>
   <223> V5 epitope tag
<400> 15
   Gly Lys Pro Ile Pro Asn Pro Leu Leu Gly Leu Asp Ser Thr
   1               5                   10
<210> 16
   <211> 9
   <212> PRT
   <213> Artificial Sequence
<220>
   <223> V5 epitope tag
<400> 16
   Ile Pro Asn Pro Leu Leu Gly Leu Asp
   1               5
<210> 17
   <211> 10
   <212> PRT
   <213> Artificial Sequence
<220>
   <223> myc tag (MYC)
<400> 17
   Glu Gln Lys Leu Ile Ser Glu Glu Asp Leu
   1               5                   10
<210> 18
   <211> 17
   <212> PRT
   <213> Artificial Sequence
<220>
   <223> CD20 rituximab-binding epitope sequence
<400> 18
   Cys Glu Pro Ala Asn Pro Ser Glu Lys Asn Ser Pro Ser Thr Gln Tyr
   1               5                   10                  15
   Cys
<210> 19
   <211> 16
   <212> PRT
   <213> Artificial Sequence
<220>
   <223> QBEnd10-binding epitope
<400> 19
   Glu Leu Pro Thr Gln Gly Thr Phe Ser Asn Val Ser Thr Asn Val Ser
   1               5                   10                  15
<210> 20
   <211> 136
   <212> PRT
   <213> Artificial Sequence
<220>
   <223> binding domain within the extracellular domain (RQR8)
<400> 20
   Cys Pro Tyr Ser Asn Pro Ser Leu Cys Ser Gly Gly Gly Gly Ser Glu
   1               5                   10                  15
   Leu Pro Thr Gln Gly Thr Phe Ser Asn Val Ser Thr Asn Val Ser Pro
               20                  25                  30
   Ala Lys Pro Thr Thr Thr Ala Cys Pro Tyr Ser Asn Pro Ser Leu Cys
           35                  40                  45
   Ser Gly Gly Gly Gly Ser Pro Ala Pro Arg Pro Pro Thr Pro Ala Pro
       50                  55                  60
   Thr Ile Ala Ser Gln Pro Leu Ser Leu Arg Pro Glu Ala Cys Arg Pro
   65                  70                  75                  80
   Ala Ala Gly Gly Ala Val His Thr Arg Gly Leu Asp Phe Ala Cys Asp
                   85                  90                  95
   Ile Tyr Ile Trp Ala Pro Leu Ala Gly Thr Cys Gly Val Leu Leu Leu
               100                 105                 110
   Ser Leu Val Ile Thr Leu Tyr Cys Asn His Arg Asn Arg Arg Arg Val
           115                 120                 125
   Cys Lys Cys Pro Arg Pro Val Val
       130                 135
<210> 21
   <211> 122
   <212> PRT
   <213> Artificial Sequence
<220>
   <223> cell surface marker protein sequence (V5-8)
<400> 21
   Met Gly Thr Ser Leu Leu Cys Trp Met Ala Leu Cys Leu Leu Gly Ala
   1               5                   10                  15
   Asp His Ala Asp Ala Gly Lys Pro Ile Pro Asn Pro Leu Leu Gly Leu
               20                  25                  30
   Asp Ser Ser Gly Gly Gly Gly Ser Pro Ala Pro Arg Pro Pro Thr Pro
           35                  40                  45
   Ala Pro Thr Ile Ala Ser Gln Pro Leu Ser Leu Arg Pro Glu Ala Cys
       50                  55                  60
   Arg Pro Ala Ala Gly Gly Ala Val His Thr Arg Gly Leu Asp Phe Ala
   65                  70                  75                  80
   Cys Asp Ile Tyr Ile Trp Ala Pro Leu Ala Gly Thr Cys Gly Val Leu
                   85                  90                  95
   Leu Leu Ser Leu Val Ile Thr Leu Tyr Cys Asn His Arg Asn Arg Arg
               100                 105                 110
   Arg Val Cys Lys Cys Pro Arg Pro Val Val
           115                 120
<210> 22
   <211> 118
   <212> PRT
   <213> Artificial Sequence
<220>
   <223> cell surface marker protein sequence (HA-8)
<400> 22
   Met Gly Thr Ser Leu Leu Cys Trp Met Ala Leu Cys Leu Leu Gly Ala
   1               5                   10                  15
   Asp His Ala Asp Ala Tyr Pro Tyr Asp Val Pro Asp Tyr Ala Ser Gly
               20                  25                  30
   Gly Gly Gly Ser Pro Ala Pro Arg Pro Pro Thr Pro Ala Pro Thr Ile
           35                  40                  45
   Ala Ser Gln Pro Leu Ser Leu Arg Pro Glu Ala Cys Arg Pro Ala Ala
       50                  55                  60
   Gly Gly Ala Val His Thr Arg Gly Leu Asp Phe Ala Cys Asp Ile Tyr
   65                  70                  75                  80
   Ile Trp Ala Pro Leu Ala Gly Thr Cys Gly Val Leu Leu Leu Ser Leu
                   85                  90                  95
   Val Ile Thr Leu Tyr Cys Asn His Arg Asn Arg Arg Arg Val Cys Lys
               100                 105                 110
   Cys Pro Arg Pro Val Val
           115
<210> 23
   <211> 119
   <212> PRT
   <213> Artificial Sequence
<220>
   <223> cell surface marker protein sequence (MYC-8)
<400> 23
   Met Gly Thr Ser Leu Leu Cys Trp Met Ala Leu Cys Leu Leu Gly Ala
   1               5                   10                  15
   Asp His Ala Asp Ala Glu Gln Lys Leu Ile Ser Glu Glu Asp Leu Ser
               20                  25                  30
   Gly Gly Gly Gly Ser Pro Ala Pro Arg Pro Pro Thr Pro Ala Pro Thr
           35                  40                  45
   Ile Ala Ser Gln Pro Leu Ser Leu Arg Pro Glu Ala Cys Arg Pro Ala
       50                  55                  60
   Ala Gly Gly Ala Val His Thr Arg Gly Leu Asp Phe Ala Cys Asp Ile
   65                  70                  75                  80
   Tyr Ile Trp Ala Pro Leu Ala Gly Thr Cys Gly Val Leu Leu Leu Ser
                   85                  90                  95
   Leu Val Ile Thr Leu Tyr Cys Asn His Arg Asn Arg Arg Arg Val Cys
               100                 105                 110
   Lys Cys Pro Arg Pro Val Val
   115
<210> 24
   <211> 59
   <212> PRT
   <213> Artificial Sequence
<220>
   <223> cell surface marker protein sequence (V5-GPI)
<400> 24
   Met Gly Thr Ser Leu Leu Cys Trp Met Ala Leu Cys Leu Leu Gly Ala
   1 5 10 15
   Asp His Ala Asp Ala Gly Lys Pro Ile Pro Asn Pro Leu Leu Gly Leu
   20 25 30
   Asp Ser Ala Ser Ser Asn Ile Ser Gly Gly Ile Phe Leu Phe Phe Val
   35 40 45
   Ala Asn Ala Ile Ile His Leu Phe Cys Phe Ser
   50 55
<210> 25
   <211> 55
   <212> PRT
   <213> Artificial Sequence
<220>
   <223> cell surface marker protein sequence (HA-GPI)
<400> 25
   Met Gly Thr Ser Leu Leu Cys Trp Met Ala Leu Cys Leu Leu Gly Ala
   1 5 10 15
   Asp His Ala Asp Ala Tyr Pro Tyr Asp Val Pro Asp Tyr Ala Ala Ser
   20 25 30
   Ser Asn Ile Ser Gly Gly Ile Phe Leu Phe Phe Val Ala Asn Ala Ile
   35 40 45
   Ile His Leu Phe Cys Phe Ser
   50 55
<210> 26
   <211> 56
   <212> PRT
   <213> Artificial Sequence
<220>
   <223> cell surface marker protein sequence (MYC-GPI)
<400> 26
   Met Gly Thr Ser Leu Leu Cys Trp Met Ala Leu Cys Leu Leu Gly Ala
   1               5                   10                  15
   Asp His Ala Asp Ala Glu Gln Lys Leu Ile Ser Glu Glu Asp Leu Ala
               20                  25                  30
   Ser Ser Asn Ile Ser Gly Gly Ile Phe Leu Phe Phe Val Ala Asn Ala
           35                  40                  45
   Ile Ile His Leu Phe Cys Phe Ser
       50                  55
<210> 27
   <211> 8
   <212> PRT
   <213> Artificial Sequence
<220>
   <223> cleavage site
<400> 27
   Asp Val Glu Met Asn Pro Gly Pro
   1               5
<210> 28
   <211> 8
   <212> PRT
   <213> Artificial Sequence
<220>
   <223> cleavage site
<400> 28
   Asp Val Glu Ser Asn Pro Gly Pro
   1               5
<210> 29
   <211> 8
   <212> PRT
   <213> Artificial Sequence
<220>
   <223> cleavage site
<400> 29
   Asp Met Glu Ser Asn Pro Gly Pro
   1               5
<210> 30
   <211> 8
   <212> PRT
   <213> Artificial Sequence
<220>
   <223> cleavage site
<400> 30
   Asp Val Glu Leu Asn Pro Gly Pro
   1               5
<210> 31
   <211> 8
   <212> PRT
   <213> Artificial Sequence
<220>
   <223> cleavage site
<400> 31
   Asp Val Glu Glu Asn Pro Gly Pro
   1               5
<210> 32
   <211> 8
   <212> PRT
   <213> Artificial Sequence
<220>
   <223> cleavage site
<400> 32
   Asp Ile Glu Leu Asn Pro Gly Pro
   1               5
<210> 33
   <211> 8
   <212> PRT
   <213> Artificial Sequence
<220>
   <223> cleavage site
<400> 33
   Asp Ile Glu Gln Asn Pro Gly Pro
   1               5
<210> 34
   <211> 8
   <212> PRT
   <213> Artificial Sequence
<220>
   <223> cleavage site
<400> 34
   Asp Ser Glu Phe Asn Pro Gly Pro
   1 5
<210> 35
   <211> 19
   <212> PRT
   <213> Artificial Sequence
<220>
   <223> cleavage sequence
<400> 35
   Leu Leu Asn Phe Asp Leu Leu Lys Leu Ala Gly Asp Val Glu Ser Asn
   1 5 10 15
   Pro Gly Pro
<210> 36
   <211> 19
   <212> PRT
   <213> Artificial Sequence
<220>
   <223> cleavage sequence
<400> 36
   Leu Leu Asn Phe Asp Leu Leu Lys Leu Ala Gly Asp Val Gln Ser Asn
   1 5 10 15
   Pro Gly Pro
<210> 37
   <211> 19
   <212> PRT
   <213> Artificial Sequence
<220>
   <223> cleavage sequence
<400> 37
   Leu Leu Asn Phe Asp Leu Leu Lys Leu Ala Gly Asp Val Glu Ile Asn
   1 5 10 15
   Pro Gly Pro
<210> 38
   <211> 19
   <212> PRT
   <213> Artificial Sequence
<220>
   <223> cleavage sequence
<400> 38
   Leu Leu Asn Phe Asp Leu Leu Lys Leu Ala Gly Asp Val Glu Phe Asn
   1               5                   10                  15
   Pro Gly Pro
<210> 39
   <211> 19
   <212> PRT
   <213> Artificial Sequence
<220>
   <223> cleavage sequence
<400> 39
   Leu Leu Asn Phe Asp Leu Leu Lys Leu Ala Gly Asp Val Glu Ser His
   1               5                   10                  15
   Pro Gly Pro
<210> 40
   <211> 19
   <212> PRT
   <213> Artificial Sequence
<220>
   <223> cleavage sequence
<400> 40
   Leu Leu Asn Phe Asp Leu Leu Lys Leu Ala Gly Asp Val Glu Ser Glu
   1               5                   10                  15
   Pro Gly Pro
<210> 41
   <211> 19
   <212> PRT
   <213> Artificial Sequence
<220>
   <223> cleavage sequence
<400> 41
   Leu Leu Asn Phe Asp Leu Leu Lys Leu Ala Gly Asp Val Glu Ser Gln
   1               5                   10                  15
   Pro Gly Pro
<210> 42
   <211> 19
   <212> PRT
   <213> Artificial Sequence
<220>
   <223> cleavage sequence
<400> 42
   Leu Leu Asn Phe Asp Leu Leu Lys Leu Ala Gly Asp Val Glu Ser Asn
   1               5                   10                  15
   Pro Gly Gly
<210> 43
   <211> 20
   <212> PRT
   <213> Artificial Sequence
<220>
   <223> 2A-like sequence
<400> 43
   Tyr His Ala Asp Tyr Tyr Lys Gln Arg Leu Ile His Asp Val Glu Met
   1               5                   10                  15
   Asn Pro Gly Pro
               20
<210> 44
   <211> 20
   <212> PRT
   <213> Artificial Sequence
<220>
   <223> 2A-like sequence
<400> 44
   His Tyr Ala Gly Tyr Phe Ala Asp Leu Leu Ile His Asp Ile Glu Thr
   1               5                   10                  15
   Asn Pro Gly Pro
               20
<210> 45
   <211> 20
   <212> PRT
   <213> Artificial Sequence
<220>
   <223> 2A-like sequence
<400> 45
   Gln Cys Thr Asn Tyr Ala Leu Leu Lys Leu Ala Gly Asp Val Glu Ser
   1               5                   10                  15
   Asn Pro Gly Pro
               20
<210> 46
   <211> 19
   <212> PRT
   <213> Artificial Sequence
<220>
   <223> 2A-like sequence
<400> 46
   Ala Thr Asn Phe Ser Leu Leu Lys Gln Ala Gly Asp Val Glu Glu Asn
   1               5                   10                  15
   Pro Gly Pro
<210> 47
   <211> 19
   <212> PRT
   <213> Artificial Sequence
<220>
   <223> 2A-like sequence
<400> 47
   Ala Ala Arg Gln Met Leu Leu Leu Leu Ser Gly Asp Val Glu Thr Asn
   1               5                   10                  15
   Pro Gly Pro
<210> 48
   <211> 20
   <212> PRT
   <213> Artificial Sequence
<220>
   <223> 2A-like sequence
<400> 48
   Arg Ala Glu Gly Arg Gly Ser Leu Leu Thr Cys Gly Asp Val Glu Glu
   1               5                   10                  15
   Asn Pro Gly Pro
   20
<210> 49
   <211> 20
   <212> PRT
   <213> Artificial Sequence
<220>
   <223> 2A-like sequence
<400> 49
   Thr Arg Ala Glu Ile Glu Asp Glu Leu Ile Arg Ala Gly Ile Glu Ser
   1 5 10 15
   Asn Pro Gly Pro
   20
<210> 50
   <211> 20
   <212> PRT
   <213> Artificial Sequence
<220>
   <223> 2A-like sequence
<400> 50
   Thr Arg Ala Glu Ile Glu Asp Glu Leu Ile Arg Ala Asp Ile Glu Ser
   1 5 10 15
   Asn Pro Gly Pro
   20
<210> 51
   <211> 20
   <212> PRT
   <213> Artificial Sequence
<220>
   <223> 2A-like sequence
<400> 51
   Ala Lys Phe Gln Ile Asp Lys Ile Leu Ile Ser Gly Asp Val Glu Leu
   1 5 10 15
   Asn Pro Gly Pro
   20
<210> 52
   <211> 20
   <212> PRT
   <213> Artificial Sequence
<220>
   <223> 2A-like sequence
<400> 52
   Ser Ser Ile Ile Arg Thr Lys Met Leu Val Ser Gly Asp Val Glu Glu
   1               5                   10                  15
   Asn Pro Gly Pro
               20
<210> 53
   <211> 20
   <212> PRT
   <213> Artificial Sequence
<220>
   <223> 2A-like sequence
<400> 53
   Cys Asp Ala Gln Arg Gln Lys Leu Leu Leu Ser Gly Asp Ile Glu Gln
   1               5                   10                  15
   Asn Pro Gly Pro
               20
<210> 54
   <211> 20
   <212> PRT
   <213> Artificial Sequence
<220>
   <223> 2A-like sequence
<400> 54
   Tyr Pro Ile Asp Phe Gly Gly Phe Leu Val Lys Ala Asp Ser Glu Phe
   1               5                   10                  15
   Asn Pro Gly Pro
               20
<210> 55
   <211> 20
   <212> PRT
   <213> Artificial Sequence
<220>
   <223> 2A-like sequence
<400> 55
   Arg Ala Glu Gly Arg Gly Ser Leu Leu Thr Cys Gly Asp Val Glu Glu
   1               5                   10                  15
   Asn Pro Gly Pro
   20
<210> 56
   <211> 57
   <212> PRT
   <213> Artificial Sequence
<220>
   <223> cleavage sequence
<400> 56
   Val Thr Glu Leu Leu Tyr Arg Met Lys Arg Ala Glu Thr Tyr Cys Pro
   1 5 10 15
   Arg Pro Leu Ala Ile His Pro Thr Glu Ala Arg His Lys Gln Lys Ile
   20 25 30
   Val Ala Pro Val Lys Gln Thr Leu Asn Phe Asp Leu Leu Lys Leu Ala
   35 40 45
   Gly Asp Val Glu Ser Asn Pro Gly Pro
   50 55
<210> 57
   <211> 40
   <212> PRT
   <213> Artificial Sequence
<220>
   <223> cleavage sequence
<400> 57
   Leu Leu Ala Ile His Pro Thr Glu Ala Arg His Lys Gln Lys Ile Val
   1 5 10 15
   Ala Pro Val Lys Gln Thr Leu Asn Phe Asp Leu Leu Lys Leu Ala Gly
   20 25 30
   Asp Val Glu Ser Asn Pro Gly Pro
   35 40
<210> 58
   <211> 33
   <212> PRT
   <213> Artificial Sequence
<220>
   <223> cleavage sequence
<400> 58
   Glu Ala Arg His Lys Gln Lys Ile Val Ala Pro Val Lys Gln Thr Leu
   1 5 10 15
   Asn Phe Asp Leu Leu Lys Leu Ala Gly Asp Val Glu Ser Asn Pro Gly
   20 25 30
   Pro
<210> 59
   <211> 24
   <212> PRT
   <213> Artificial Sequence
<220>
   <223> cleavage sequence
<400> 59
   Ala Pro Val Lys Gln Thr Leu Asn Phe Asp Leu Leu Lys Leu Ala Gly
   1 5 10 15
   Asp Val Glu Ser Asn Pro Gly Pro
   20
<210> 60
   <211> 347
   <212> DNA
   <213> Artificial Sequence
<220>
   <223> PiggyBAC 5' Terminal Repeat
<400> 60
   cattctagat taaccctaga aagatagtct gcgtaaaatt gacgcatgca ttcttgaaat 60
   attgctctct ctttctaaat agcgcgaatc cgtcgctgtg catttaggac atctcagtcg 120
   ccgcttggag ctcccgtgag gcgtgcttgt caatgcggta agtgtcactg attttgaact 180
   ataacgaccg cgtgagtcaa aatgacgcat gattatcttt tacgtgactt ttaagattta 240
   actcatacga taattatatt gttatttcat gttctactta cgtgataact tattatatat 300
   atattttctt gttatagata tcaactagaa tgctagcatg ggcccat 347
<210> 61
   <211> 243
   <212> DNA
   <213> Artificial Sequence
<220>
   <223> PiggyBAC 3' Terminal Repeat
<400> 61
   agttttgtta ctttatagaa gaaattttga gtttttgttt ttttttaata aataaataaa      60
   cataaataaa ttgtttgttg aatttattat tagtatgtaa gtgtaaatat aataaaactt      120
   aatatctatt caaattaata aataaacctc gatatacaga ccgataaaac acatgcgtca      180
   attttacgca tgattatctt taacgtacgt cacaatatga ttatctttct agggttaatc      240
   tag                                                                     243